# EUROPEAN PATENT SPECIFICATION

(11) **EP 1 422 230 B1**
(45) Date of publication and mention of the grant of the patent: **26.12.2007**
(21) Application number: 02258083.1
(22) Date of filing: 25.11.2002
(51) Int. Cl.: C07D 489/12, A61K 31/485, A61P 25/04

(54) **Novel ester derivatives of buprenorphine and their preparation processes, and long acting analgestic pharmaceutical compositions**
Buprenorphinesterderivate, Verfahren zu ihrer Herstellung, und langwirksame analgetische Arzneimittel
Dérivés estérifiés de buprenorphine, procédé pour leur préparation, et médicaments analgésiques à action prolongée

(43) Date of publication of application: 26.05.2004
(73) Proprietor: Chi Mei Foundation Medical Center, Yung Kang City, Tainan (TW)
(72) Inventor: Wang, Jhi-Joung, Yung Kang City, Tainan (TW)
(74) Representative: Eddowes, Simon

(56) References cited:
- WO-A-96/16063
- WO-A-96/28451
- STINCHCOMB A L ET AL: "Permeation of buprenorphine and its 3-alkyl-ester prodrugs through human skin" PHARMACEUTICAL RESEARCH, vol. 13, no. 10, October 1996 (1996-10), pages 1519-1523, XP008016388
- STINCHCOMB A L ET AL: "A solubility and related physicochemical property comparison of buprenorphine and its 3-alkyl esters" PHARMACEUTICAL RESEARCH, vol. 12, no. 10, October 1995 (1995-10), pages 1526-1529, XP008016370
- HUSSAIN M A ET AL: "Improvement of the oral bioavailability of naltrexone in dogs: a prodrug approach" JOURNAL OF PHARMACEUTICAL SCIENCES, vol. 76, no. 5, May 1987 (1987-05), pages 356-358, XP002238857
- EVERHART E T ET AL: "Subnanogram-concentration measurement of buprenorphine in human plasma by electron-capture capillary gas chromatography: application to pharmacokinetics of sublingual buprenorphine" CLINICAL CHEMISTRY, vol. 43, no. 12, 1 December 1997 (1997-12-01), pages 2292-22302, XP002238858
- DATABASE CAPLUS [Online] CHEMICAL ABSTRACTS SERVICE, COLUMBUS, OHIO, US; retrieved from STN Database accession no. 1985:608220 XP002238859 & YASHIKI M ET AL: "Dual mass spectrometry of trifluoroacetyl derivatives of opioid bases" GC-MS NEWS, vol. 13, no. 4, 1985, pages 101-106,
- DATABASE WPI Week 199539, Derwent Publications Ltd., London, GB; Class B02, AN 1995-299497 & JP 7 196 510 A (NIPPON SEIYAKU KK) 01 August 1995
- IMOTO H. ET AL: 'Transdermal prodrug concepts: permeation of buprenorphin and its alkyl esters through hairless mouse skin and influence of vehicles' BIOLOGICAL & PHARMACEUTICAL BULLETIN vol. 19, no. 2, February 1996, pages 263 - 267, XP008060357

## Description

### BACKGROUND OF THE INVENTION

### 1. Field of the Invention

The present invention relates to novel ester derivatives of buprenorphine, in particular buprenorphine monocarboxylic ester derivatives and dibuprenorphine dicarboxylic ester derivatives, which exert a longer analgesic effect as compared to buprenorphine hydrochloride. The invention also relates to processes for preparing the novel ester derivatives of buprenorphine, and long-acting analgesic pharmaceutical compositions containing a compound selected from buprenorphine base and the novel ester derivatives of buprenorphine.

### 2. Description of the Related Art

Prolonged analgesia is particularly desirable in patients suffering from moderate to severe pain, such as postoperative pain and cancer pain. Currently, local anesthetics, weak analgesics and potent analgesics are used in this field, but they are all short-acting drugs.

Local anesthetics, e.g., xylocaine or bupivacaine, relieve some types of pain but they can only be applied to restricted areas. In addition, local anesthetics are short-acting and exhibit a duration of action normally no more than 6 hours even when introduced intrathecally. Therefore, local anesthetics are not satisfactory for the relief of acute and severe pain caused by cardiac, pulmonary, abdominal, orthopedic and obstetrical surgery, severe burn injury, and terminal stage of cancer.

Weak analgesics, such as acetaminophen and nonsteroidal anti-inflammatory agents (NSAID), relieve pain of only low intensity, such as pain due to headache or toothache, but they do not help in the case of severe pain.

For the pain of high intensity and widespread in origin, potent analgesics, such as morphine, meperidine and fentanyl, are used. They interact with specific opioid receptors (i.e. *µ* receptors) in the central nervous system (CNS) and exhibit potent analgesic activity. However, all the opioid analgesics exhibit common disadvantages (Hayes, A. G. et al., Br. J. Pharmacol., Vol 79, 731, 1983)*.* The most unwanted problem associated with the long-term use of these potent analgesics is the incidence of addiction. In addition, these potent analgesics may induce severe respiratory depression in patients with poor respiratory function. Moreover, these potent analgesics exhibit a relatively short duration of action, i.e. 3-5 hours. Even when they are administered intrathecally, they fail to provide a duration of action that lasts for a period of more than 24 hours. In addition, if a larger dose of such an agent, e.g. morphine of 0.5-1.0 mg/does, is administered intrathecally to provide a prolonged analgesic effect, fatal respiratory depression is likely to occur (Baxter, A. D. et al., Can. J. Anesth., Vol 36, 503, 1989*).*

Buprenorphine, the chemical name of which is (5*α,* 7α (S)-17- cyclopropyl-methyl)- α -(1,1-dimethylethyl)-4,5-epoxy- 18,19-dihydro-3-hydroxy-6-methoxy- α -methyl-6,14-ethenomorphinan-7-methanol, is known to be an opioid partial agonist having a potent analgesic effect . Buprenorphine (in free base form), the molecular weight of which is 467.7, is represented by the following formula (A):

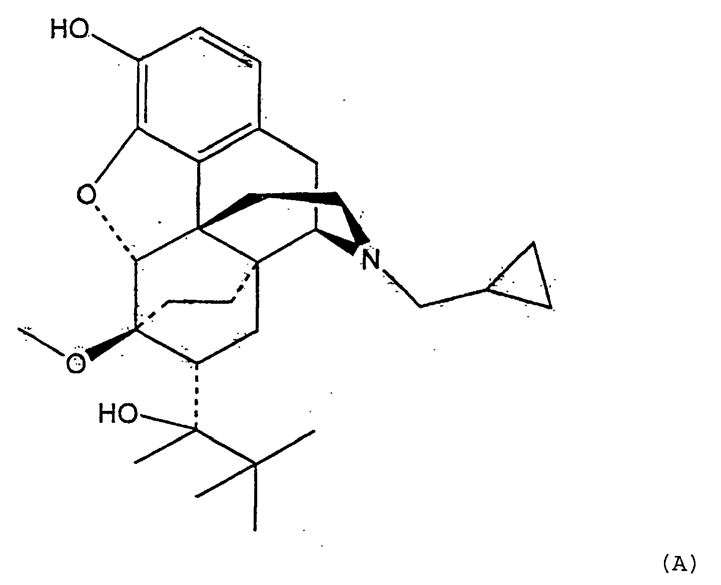

Buprenorphine shares many of the actions of opioid agonists, such as analgesia. The lack of κ agonism accounts for buprenorphine's freedom from the dysphoric and psychotomimetic effects often seen with agonist/antagonist drugs. Other studies suggest that the opioid antagonist effect of buprenorphine may be mediated via an interaction with δ opioid receptors.

Like other potent opioid agonists, buprenorphine produces potent dose-related analgesia. While the exact mechanism has yet to be fully known, the analgesic effect of buprenorphine appears to arise from a high affinity of buprenorphine for *µ* opioid receptors in the central nerve system. In addition, buprenorphine may alter the pain threshold (threshold of afferent nerve endings to noxious stimuli). On a weight basis, the analgesic potency of parenteral buprenorphine appears to be about 25 to 50 times as that of morphine, about 200 times as that of pentazocine, and about 600 times as that of meperidine.

Buprenorphine provides several therapeutic advantages in many patients as compared to opioid agonists (e.g., morphine) and mixed agonists-antagonists (e.g., pentazocine, butorphanol, nalbuphine) . For example, unlike the mixed agonists-antagonists, buprenorphine has less psychotomimetic effects. When compared with agonists (e. g. , morphine and fentanyl), buprenorphine brings about a relatively low risk of respiratory depression.

Buprenorphine has a lesser abuse liability as compared to full agonist opioids. Although infrequent, however, buprenorphine may cause limited physical dependence, and signs and symptoms of mild withdrawal may appear subsequent to discontinuance of a prolonged therapy with buprenorphine alone. Due to buprenorphine's slow dissociation from the *µ* receptor, elimination of buprenorphine from the CNS is prolonged subsequent to abrupt discontinuance. As a consequence, signs and symptoms due to an acute withdrawal of buprenorphine are less intense than those produced by morphine and occur at a later time.

Buprenorphine, which was disclosed in US 3,433,791 (1968), is sold under the trademarks Buprenex (Morton-Norwich) and Temgesic (Reckitt and Colman), and has been used principally for the management of pain due to surgery, cancer, accidental trauma and myocardial infarction. Buprenorphine has also been used in the detoxification treatment of heroin addicts due to its opioid partial agonist properties (Bickel, W. K., et al., Chem. Pharmacol. Ther. (1988), 43 (1) :72-78*;* and Fudala, P. J. , et al . , Clin. Pharmacol. Ther. (1990), 47 (4):525-534*)*. Buprenorphine has been administered commonly by intramuscular injection or intravenous injection, but the duration of action is only 6-8 hours.

A long-acting analgesic effect is particularly desirable in patients suffering from acute or chronic pain. These pains may last from days to months. For example, acute pain, such as postoperative pain, traumatic pain, and burn pain, may last 4-6 days; chronic pain, such as nonmalignant pain and cancer pain, may last from several weeks to a few months.

In order to control or prolong the duration of action of a target drug during clinical use, there have been developed certain pharmaceutical preparations. In regard to buprenorphine, due to the high level of crystallinity as reflected in the melting point of its free base, 218 °C, buprenorphine is not likely to be a good candidate for transdermal drug delivery by itself. Utilization of hydrochloride salt, along with a permeation enhancer, has been offered as one solution to obtain sufficient skin permeation of buprenorphine for analgesic purposes. For example, US 6,004,969 disclosed a transdermal delivery preparation of buprenorphine, which consisted of buprenorphine or the hydrochloride salt thereof, pure components from Chinese herbs as transdermal penetration enhancers, and other excipients necessary for transdermal preparations.

A prodrug strategy is another possibility. Stinchcomb et al. reported in Pharm. Res. (1995), 12 (10): 1526-1529 six alkyl ester prodrugs of buprenorphine, the synthesis of which started from buprenorphine hydrochloride and involved the reaction of buprenorphine free base, an acid anhydride and 4-dimethylaminno pyridine in the presence of dimethylformamide (DMF) as solvent. The physicochemical properties, including hexane solubility, of the six alkyl ester prodrugs of buprenorphine were compared with those of buprenorphine HCl and buprenorphine free base. Stinchcomb *et al.* further investigated the permeation of buprenorphine and its C₂-C₄ alkyl ester prodrugs through hairless mouse skin and human skin, in which light mineral oil was used as a vehicle in the tested formulations for the permeation experiments (Hirofumi Imoto et al., Biol. Pharm. Bull. (1996), 19 (2) : 263-267*;* and Stinchcomb et al., Pharm. Res. (1996), 13 (10) : 1519-1523*)*

Furthermore, an oil-in-water type slow releasing fat emulsion of an opioid analgesic such as buprenorphine, pentazocine and morphine is disclosed in AN 1995-299497, Database WP1 Derwent Publications Ltd, London, GB. Additionally, patent WO 96/28451 is directed to morphine-3-esters.

However, no injectable long-acting dosage form of buprenorphine suitable for therapeutic use has been described heretofore. In addition, it is still desirable to develop new derivatives of buprenorphine which effectively extends the duration of action of buprenorphine in vivo.

### SUMMARY OF THE INVENTION

Therefore, an object of this invention is to provide novel ester derivatives of buprenorphine which have a longer duration of action and same activity as buprenorphine HCl so that they may be used in the treatment of living subjects including humans suffering from severe pain.

In one aspect, the present invention provides an analgesic pharmaceutical composition comprising
a) a therapeutically effective amount of
   i) a buprenorphine monocarboxylic ester derivative of formula (I) : 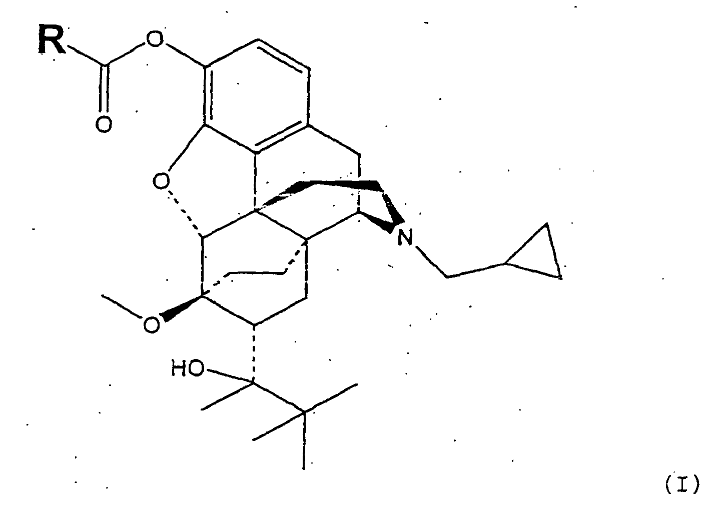 wherein R is selected from the group consisting of a straight-chain or branched saturated or unsaturated aliphatic group optionally substituted with an aryl group, and an aryl group optionally substituted with a straight-chain or branched saturated or unsaturated aliphatic group;
      with the proviso that R is not selected from methyl, ethyl, propyl, n-butyl, n-pentyl, n-hexyl and isopropyl, or
   ii) a dibuprenorphine dicarboxylic ester derivative according to the invention, and
b) a pharmaceutically acceptable oil carrier.

In a further aspect, the present invention provides new dibuprenorphine dicarboxylic ester derivatives of formula (II) 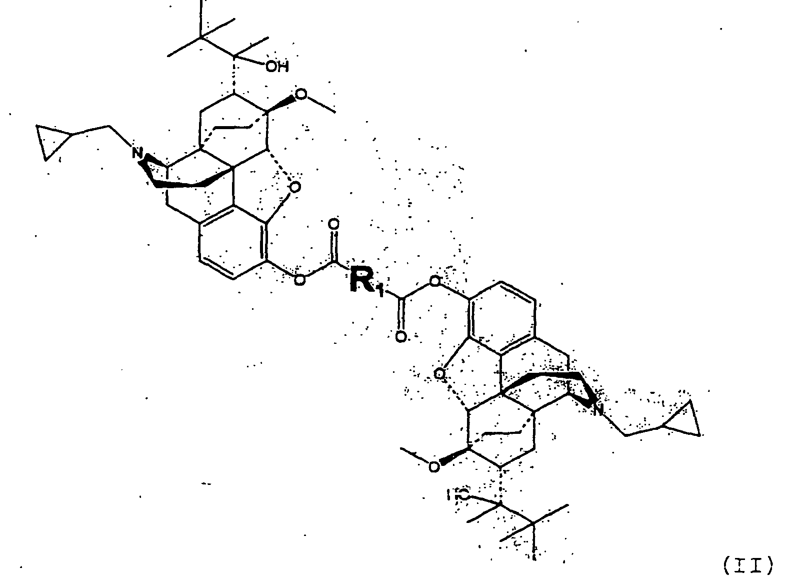 wherein R₁ is a divalent moiety of a saturated or unsaturated aliphatic group having 1 to 40 carbon atoms optionally substituted with a phenyl group. Preferably, R₁ is an alkylene group having 1 to 40 carbon atoms, and preferably 1 to 20 carbon atoms. Preferably the dibuprenorphine dicarboxylic ester derivative is dibuprenorphine pimelate or dibuprenorphine sebacoyl ester.

The method of preparing the dibuprenorphine dicarboxylic ester derivative of formula (II) includes the steps of:
(i') treating buprenorphine HCl or base with trimethylamine in the presence of methylene chloride; and
(ii) adding to the mixture from step (i') a compound of formula R₁ (COOH) ₂, or an acid anhydride or an acid chloride thereof, in the presence of methylene chloride, wherein R₁ in the formula R₁ (COOH)₂ is a divalent moiety of a saturated or unsaturated aliphatic group having 1 to 40 carbon atoms and optionally substituted with a phenyl group.

In an even further aspect, the present invention provides use of a composition comprising a therapeutically effective amount of buprenorphine base and an injectable oil and substantially free of water in the manufacture of an injectable medicament for providing a prolonged analgesia to a human or animal. Also provided is the use of dibuprenorphine dicarboxylic ester derivative according to the invention for the preparation of an injectable medicament for providing prolonged analgesia to an animal or human.

In an even further aspect, the invention provides the use of a combination of (i) a buprenorphine monocarboxylic ester derivative, or a dibuprenorphine dicarboxylic ester derivative according to the invention; and (ii) a pharmaceutically acceptable oil vehicle, for the preparation of a medicament for providing prolonged analgesia to an animal or human.

### BRIEF DESCRIPTION OF THE DRAWING

The above and other features and advantages of the present invention will become apparent in the following detailed description of the preferred embodiments with reference to the accompanying drawing, of which:
Figure 1 shows the ¹H-NMR spectrum chart of buprenorphine enanthate;
Figure 2 shows the mass spectrum chart of buprenorphine enanthate;
Figure 3 shows the UV chart of buprenorphine enanthate;
Figure 4 shows the IR spectrum chart of buprenorphine enanthate;
Figure 5 shows the ¹H-NMR spectrum chart of buprenorphine decanoate;
Figure 6 shows the mass spectrum chart of buprenorphine decanoate;
Figure 7 shows the UV chart of buprenorphine decanoate,
Figure 8 shows the IR spectrum chart of buprenorphine decanoate;
Figure 9 shows the mass spectrum chart of buprenorphine pivalate;
Figure 10 shows the UV chart of buprenorphine pivalate;
Figure 11 shows the IR spectrum chart of buprenorphine pivalate;
Figure 12 shows the UV chart of buprenorphine palmitate;
Figure 13 shows the IR spectrum chart of buprenorphine palmitate;
Figure 14 shows the IR spectrum chart of dibuprenorphine pimelate;
Figure 15 shows the UV chart of dibuprenorphine pimelate;
Figure 16 shows the ¹H-NMR spectrum chart of dibuprenorphine sebacoyl ester;
Figure 17 shows the UV chart of dibuprenorphine sebacoyl ester;
Figure 18 shows the IR spectrum chart of dibuprenorphine sebacoyl ester;
Figure 19 shows the dose response of intramuscularly administered buprenorphine hydrochloride in rats;
Figures 20-A and 20-B show the dose responses of intramuscularly and subcutaneously administered buprenorphine base in rats, respectively;
Figures 21-A to 21-E show the analgesic effects of five intramuscularly administered buprenorphine monocarboxylic ester derivatives of this invention in rats, respectively;
Figure 22 shows the dose response of intramuscularly administered buprenorphine propionate in rats; and
Figures 23-A and 23-B show the analgesic effects of two intramuscularly administered dibuprenorphine dicarboxylic ester derivatives of this invention in rats, respectively.

### DETAILED DESCRIPTION OF THIS INVENTION

A parenteral solution of buprenorphine hydrochloride (0.3 mg buprenorphine/mL) is commercially available as Buprenex.RTM. (Reckitt & Colman) for intramuscular and intravenous administration. The usual intramuscular or intravenous dose for adults at age over 13 is 0.3 mg every 6 to 8 hours as needed for the relief of moderate to severe pain. The pediatric dose for patients aged 2 to 12 is 2-6 µg/kg of body weight every 4-6 hours. The mean duration of analgesia is generally six hours following a single intramuscular or intravenous dose of 0.2 to 0.3 mg or 2 to 4 µg/kg of body weight.

The pharmacokinetics of buprenorphine administered parenterally and sublingually are known. Intravenous administration of a single dose of about 0.3 mg of buprenorphine has been shown to provide mean peak plasma drug concentrations of about 18 ng/mL which occurred within about 2 minutes. Plasma concentrations declined to about 9 and about 0 .4 ng/mL after about 5 minutes and about 3 hours, respectively. Following an intramuscular administration of a second 0.3-mg dose 3 hours after the initial intravenous dose, mean peak plasma buprenorphine concentrations of about 3.6 ng/mL occurred within about 2 to about 5 minutes and declined to about 0.4 ng/mL after about 3 hours. Approximately 10 minutes after administration, plasma concentrations of buprenorphine are similar following intravenous or intramuscular injection. It has been previously reported that a usual sublingual analgesic dose of buprenorphine is 0.2 to 0.4 mg every 8 hours (see, e.g. , Kuhlman, JJ et al., J. Analyt. Toxicol. (1996), 20 (10) : 369-378*)*. For a 0.4 mg sublingual dose, the Cₘₐₓ was reported as 0.5±0.06 ng/mL; the Tₘₐₓ was reported as 210±40 min; and a systemic availability of 57.7±6% was also reported.

Buprenorphine is almost completely metabolized in the liver, principally by N-dealkylation, to form norbuprenorphine (N-dealkylbuprenorphine) . Buprenorphine and norbuprenorphine also undergo conjugation with glucuronic acid. Like the metabolites of other opioid agonists, norbuprenorphine may have weak analgesic activities. However, studies to determine the analgesic activity of the metabolites of buprenorphine have not been performed. Buprenorphine and its metabolites are excreted principally in feces via biliary elimination and also in urine. Buprenorphine is excreted in feces mainly as unchanged drug. Small amounts of norbuprenorphine are also excreted in feces. The drug and its metabolites are believed to undergo enterohepatic circulation. Norbuprenorphine appears to be excreted principally in urine at a slower rate than the parent drug. Total plasma clearance of buprenorphine is reported to be approximately 0.28 L/minute in conscious postoperative patients. Limited data indicate that there is considerable interindividual variability in buprenorphine pharmacokinetics in children. However, clearance of the drug appears to be faster in children (e.g. , those 5 to 7 years of age) as compared to that in adults. Optimal dosing interval of buprenorphine may have to be decreased in pediatric patients.

In view of the aforesaid, the Applicant endeavored to prolong the duration of action of buprenorphine and synthesized novel ester derivatives of buprenorphine, which have been proved to have the same analgesic activity as that of buprenorphine HCl. Sustained release pharmaceutical compositions comprising a compound selected from buprenorphine base and the novel ester derivatives of buprenorphine according to this invention were further developed. These compositions were demonstrated to exhibit long-acting analgesic effects of several days with a relatively rapid onset of action (within 2 hours).

Esterification of the phenol group on carbon atom 3 of the morphine ring (the basic structure of morphine, buprenorphine, nalbuphine and the like) enables the esterified derivatives to have the following characteristics: (1) increased lipophilicity; (2) low affinity to morphine receptors; (3) the side effects are decreased, but released parent drugs maintain the same pharmacological activity; and (4) the effect and safety of esterified derivatives and mother compounds remain the same *(*Broekkamp CL et al., J. Pharm. Pharmacol, 1988, 40: 434-7*).*

The present invention provides new pharmaceutical compositions containing buprenorphine monocarboxylic ester derivatives of formula (I): 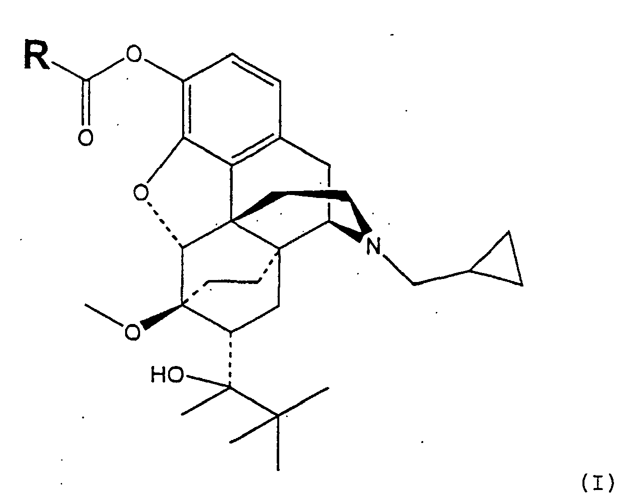 wherein R is selected from the group consisting of a straight-chain or branched saturated or unsaturated aliphatic group optionally substituted with an aryl group, and an aryl group optionally substituted with a straight-chain or branched saturated or unsaturated aliphatic group;
with the proviso that R is not selected from methyl, ethyl, propyl, n-butyl, n-pentyl, n-hexyl and isopropyl.

Preferably, R is an alkyl group optionally substituted with a phenyl group.

Preferably, R is an alkyl group having 2 to 40 carbon atoms, and more preferably, an alkyl group having 5 to 20 carbon atoms.

Preferably, R is selected from the group consisting of a straight-chained alkyl group optionally substituted with a phenyl group, a branched alkyl group optionally substituted with a phenyl group, a phenyl group optionally substituted with a straight-chain aliphatic group, and a phenyl group optionally substituted with a branched aliphatic group.

In a preferred embodiment of this invention, R is an alkyl moiety derived from a fatty acid of formula RCOOH. More preferably, R represents an alkyl group optionally substituted with a phenyl and having 2 to 40 (preferably 5 to 20) carbon atoms.

The preferred buprenorphine monocarboxylic esters according to this invention are prepared from buprenorphine and a carboxylic acid selected from the group consisting of: propionic acid, benzoic acid, enanthic acid, n-valeric acid, pivalic acid, decanoic acid; saturated fatty acids, such as lauric acid, palmitoyl acid, stearic acid, arachidic acid and cerotic acid, etc.; and unsaturated fatty acid, such as oleic acid, linolenic acid, undecylenic acid and cinnamic acid, etc.

Preferably, the buprenorphine monocarboxylic ester derivative according to this invention is selected from buprenorphine pivalate, buprenorphine benzoate, buprenorphine decanoate and buprenorphine palmitate.

The present invention also provides new dibuprenorphine dicarboxylic ester derivatives of formula (II): 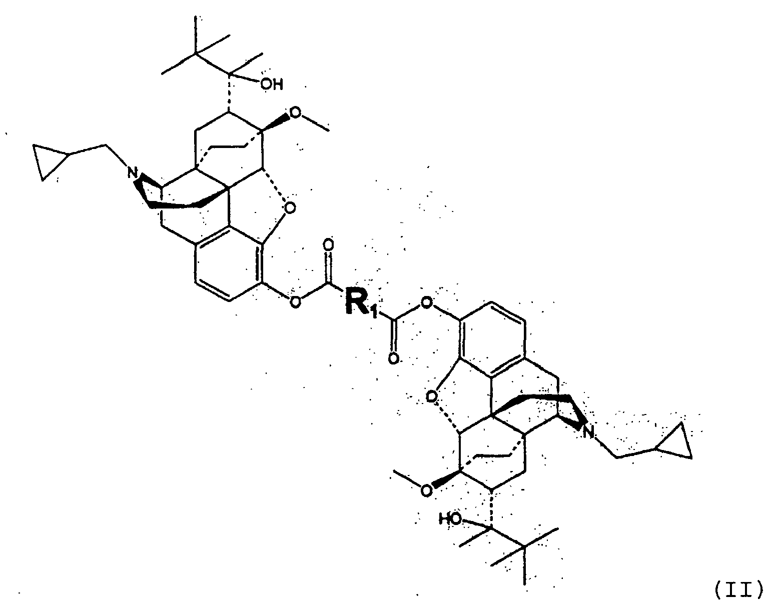 wherein R₁ is a divalent moiety of a saturated or unsaturated aliphatic group optionally substituted with a phenyl group.

Preferably, the aliphatic group is selected from a straight-chain alkyl group, a branched alkyl group, a straight-chain alkyl group substituted with a phenyl group and a branched alkyl group substituted with a phenyl group. The aliphatic group has 1 to 40 carbon atoms, and more preferably, 1 to 20 carbon atoms.

Preferably, R₁ is an alkylene group having 1 to 40 (more preferably, 3 to 20) carbon atoms.

Preferably, the dibuprenorphine dicarboxylic esters according to this invention are prepared frombuprenorphine and a C₅-C₂₀ aliphatic dicarboxylic acid.

Preferably, the dibuprenorphine dicarboxylic ester derivative according to this invention is selected from dibuprenorphine pimelate and dibuprenorphine sebacoyl ester.

Buprenorphine base was prepared from its HCl salt. A given amount of commercial buprenorphine HCl was dissolved in water, followed by the addition of a saturated solution of Na₂CO₃, to precipitate buprenorphine base. The precipitate was then filtered and washed several times with cold deionized water to remove excess Na₂CO₃. The white residue was then dried overnight in air. The dried residue was added into a water:ethanol (80:20) mixture, and heated to 60°C, to dissolve the free base, followed by immediate filtration. Upon cooling, the buprenorphine base crystallized. The crystalline product was then filtered and dried under a gentle stream of nitrogen. The purity of the base was checked by the melting point and HPLC assay. The melting point of the base was 209°C, virtually the same as reported in literature. The purity of the base as determined by HPLC assay was 99%.

The buprenorphine monocarboxylic ester derivative of formula (I) can be prepared by a process comprising the following steps:
(i) treating buprenorphine HCl or base with trimethylamine in the presence of methylene chloride; and
(ii) adding to the mixture from step (i) a compound of formula RCOOH, or an acid anhydride or an acid chloride thereof, in the presence of methylene chloride, wherein R in the formula RCOOH is selected from the group consisting of a straight-chain or branched saturated or unsaturated aliphatic group optionally substituted with an aryl group, and an aryl group optionally substituted with a straight-chain or branched saturated or unsaturated aliphatic group.

Preferably, an aliphatic carboxylic acid having 1-40 (more preferably, 5-20) carbon atoms, or an acid anhydride or an acid chloride thereof, is used in the above step (ii) . More preferably, a saturated C₅-C₂₀ aliphatic carboxylic acid is used in the above step (ii).

In a preferred embodiment of this invention, heptanoyl chloride is used in the above step (ii).

In another preferred embodiment of this invention, decanoyl chloride is used in step (ii).

In a further preferred embodiment of this invention, pivaloyl chloride is used in step (ii).

In another further preferred embodiment of this invention, hexadecanoyl chloride is used in step (ii).

In yet another preferred embodiment of this invention, benzoyl chloride is used in step (ii).

The buprenorphine polyester derivative of formula (II) according to this invention can be prepared by a process comprising the following steps:
(i') treating buprenorphine HCl or base with trimethylamine in the presence of methylene chloride; and
(ii') adding to the mixture from step (i') a compound of formula R₁ (COOH) 2' or an acid anhydride or an acid chloride thereof, in the presence of methylene chloride, wherein R₁ in the formula R₁ (COOH) is a divalent moiety of a saturated or unsaturated aliphatic group optionally substituted with a phenyl group.

Preferably, an aliphatic dicarboxylic acid having 3-40 (more preferably, 5-20)carbon atoms, or an acid anhydride or an acid chloride thereof, is used in the above step (ii'). More preferably, a saturated C₅-C₂₀ aliphatic dicarboxylic acid is used in the above step (ii').

In a preferred embodiment of this invention, heptanedioyl chloride is used in the above step (ii').

In another preferred embodiment of this invention, sebacoyl acid is used in the above step (ii').

To synthesize the buprenorphine ester derivatives represented by formula (I) and formula (II), respectively, buprenorphine HCl or base was dissolved in methylene chloride, followed by the addition of a solution of triethylamine in methylene chloride. To the mixture was added dropwise a solution of a compound of formula R (COOH)₂ or formula R₁(COOH)₂ in methylene chloride.

Upon completion of esterification, the resultant product was purified by passing through a silical gel column, and a buprenorphine ester derivative represented by formula (I) or formula (II) was obtained.

As an alternative, the buprenorphine ester derivatives of this invention may be obtained by the general method of preparing esters from alcohols or phenols, for instance, by reacting the hydroxyl group of buprenorphine with acid chlorides, acid anhydrides, esters or sulfonyl chlorides of the compound formula R (COOH)₂ or formula R₁(COOH)₂.

The buprenorphine ester derivatives synthesized by the methods described above have been identified by nuclear magnetic resonance (NMR), infrared (IR) and ultraviolet (UV) spectroscopy, gas chromatography/mass spectrometry (CG/MS), and elementary analysis.

The buprenorphine ester derivatives may be formulated into different dosage forms as desired.

In general, to achieve the long acting therapeutic efficacy of a target, various dosage forms may be prepared in which the target drug is esterified or dissolved in an oil vehicle to form a parenteral formulation, so that upon being administered to the human or animal body, the release rate of the target drug may slow down due to the influence of some factors, such as the increased solubility of the target drug in oil. In these cases, the dosing interval of the target drug can be set longer by virtue of the prolonged duration of action thereof.

Gelders reported in Int. Clin. Psychophacol (1986), volume 1, page 1*,* and Hinko, C.N. et al. reported in Neuropharmacology (1998), volume 27, page 475*,* the formation of a controlled-release dosage form of haloperidol decanoate in injectable oil, such as sesame oil or soybean oil, the analgesic effect of which was prolonged due to the extended dosing interval from 2 to 4 times a day to 1 to 2 times a month.

Norman T.R. reported in Int. Clin. Psychopharmacol. (1987), Volum 2, pp. 299-305*,* the preparation of fluphenazin decanoate from fluphenazin. Hinko, C.N. reported in Neuropharmacology (1988), volume 27, pp. 475-483*,* the preparation of an ester of nipectic acid. BroekkampC.L. reported in J. Pharm. Pharmacol. (1988), Vol. 40, pp. 434-437*,* the preparation of nicotinoyl morphine ester from morphine. Joshi, J.V. et al. reported in Steroids (1989), volume 53, pp. 751-761*,* a precursor preparation of northisterone enenthate, which could be set with a longer dosing interval of up to two months.

However, due to unknown factors present in nature, quick release of a target drug from an oil vehicle could sometimes occur. For instance, the release of testosterone from the intramuscular administration of a testosterone suspension was found to be quick *(*Tanaka, T., Chem. Pharm. Bull. (1974), Vol. 22, pp. 1275-1284*).* Titulaer, H.A.C. reported the addition of artemisinin in parenteral oil to form various dosage forms for intramuscular, intravenous, oral or rectal administration. However, the drug was released quickly from such dosage forms *(*J. Pharm. Pharmacol. (1990), Vol. 42, pp. 810-813*) .* Zuidema, Z. et al. reported in International J. of Pharmaceutics (1994), Vol. 105, pp. 189-207*,* that the release rate and extent of dosage forms for parenteral administration are very erratic and variable.

According to the aforementioned studies, a dosage form which contains a pharmaceutical composition suspended, or dissolved in an oil vehicle does not certainly exhibit a longer duration of therapeutic effect. In general, any attempt to add a target drug into an oil vehicle for the purpose of obtaining long-acting dosage forms need to take into account the physical solubility, stability, and release rate of the target drug from such vehicle.

In view of the aforesaid, in order to achieve the goal of extending the duration of action of buprenorphine, the Applicant provided in this application an analgesic pharmaceutical preparation which comprises an injectable oil containing a compound selected from buprenorphine base, a buprenorphine monocarboxylic ester derivative of formula (I), and a dibuprenorphine dicarboxylic ester derivative of formula (II) as described above, in admixture with an injectable oil, and optionally a pharmaceutically acceptable excipient. This preparation permits the compound contained therein to have a longer duration of action in relieving pain.

The suitable injectable oil for use in this invention, which acts as a vehicle for injection preparations, includes, e.g. sesame oil, soybean oil, castor oil, cotton seed oil, peanut oil or ethyl ester of peanut oil, or a combination thereof. The injectable oil preparations can be administered via the intramuscular and subcutaneous routes.

The present invention also provides a process for preparing a long-acting dosage form for buprenorphine base and the novel buprenorphine ester derivatives according to this invention, in which buprenorphine base, or a buprenorphine ester derivative of formula (I) or (II) as described above, is admixed with an injectable oil vehicle, optionally with the addition of pharmaceutically acceptable excipient(s) commonly employed in the manufacture of medicaments, to thereby form a controlled-release dosage form.

According to this invention, the pharmaceutically acceptable excipient, if present, may be selected from benzyl alcohol or chlorobutanol or a combination thereof.

The long-acting parenteral dosage form of this invention can be administered once for several days. Even when long-acting parenteral dosage form of this invention was administered with a larger amount, the occurrence of undesired effects was minimized.

As described above, the advantages of the present pharmaceutical composition include a prolonged duration of action, a rapid onset of action (within 2 hours) and safety that should improve therapeutic quality. The present pharmaceutical composition can be set with a dosing interval of several days instead of 6-8 hours for patients suffering from pain.

### Examples:

The following examples are given for the purpose of illustration only and are not intended to limit the scope of the present invention.

The following Table 1 shows the chemical structures of the preferred buprenorphine ester derivatives according to this invention .

**Table 1.The molecular structures of buprenorphine HCl, buprenorphine base and the ester derivatives of according to this invention**

| Compound | Molecular structure |
|---|---|
| Buprenorphine HCl | Bup • HCl |
| Buprenorphine base | Bup |
| Buprenorphine propionate | 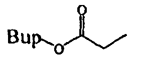 |
| Buprenorphine pivalate | 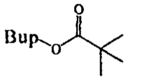 |
| Buprenorphine benzoate | 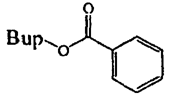 |
| Buprenorphine enanthate | 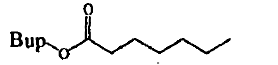 |
| Buprenorphine decanoate | 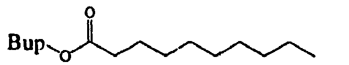 |
| Buprenorphine palimitate | 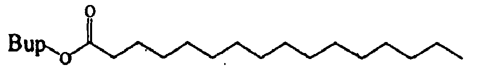 |
| Dibuprenorphine pimelate | 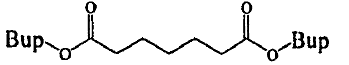 |
| Dibuprenorphine sebacoyl ester | 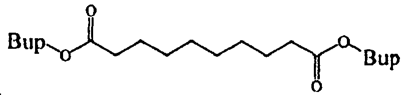 |

| | |
|---|---|
| Bup: Buprenorphine The buprenorphine ester derivatives listed in Table 1 can be synthesized by suitable known methods, such as those disclosed in U.S. Patent Nos. 5,750,534 and 6,225,321. | |

### Synthesis Ex. 1 Preparation of buprenorphine enanthate

75 mL of methylene chloride (Mallinckrodt, Baker, U.S.A.) and 0.01 mole of buprenorphine HCl or base were added into a 250-mL round-bottomed flask which was placed in an ice bath for cooling. The mixture was stirred and 20 mL of methylene chloride containing 0.03 mole of triethylamine (Sigma, MO, U.S.A.) was gradually added thereto. With rapid stirring, another 20 mL of methylene chloride containing 0.011 mole of heptanoyl chloride (Aldrich, Milwaukee, U. S .A) was added dropwise. Thereafter, the mixture was stirred at room temperature for 1 hour. 20 mL of a 10% sodium carbonate solution was then added to neutralize the residual acid and remove water-soluble impurities. Sodium sulfate was used to dehydrate the solution. After drying under vacuum, the title compound, i.e. buprenorphine enanthate, was obtained. The product was purified by column chromatography.

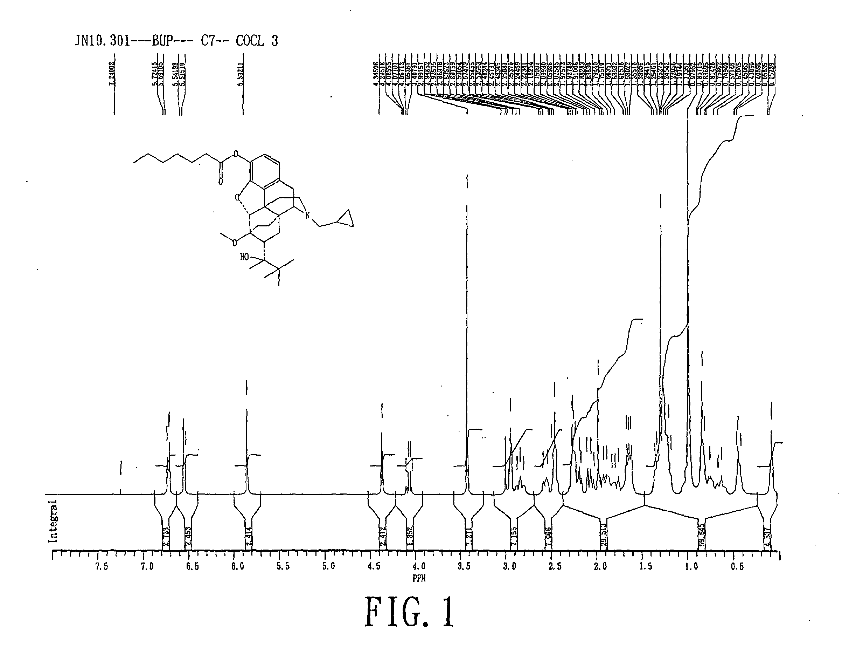

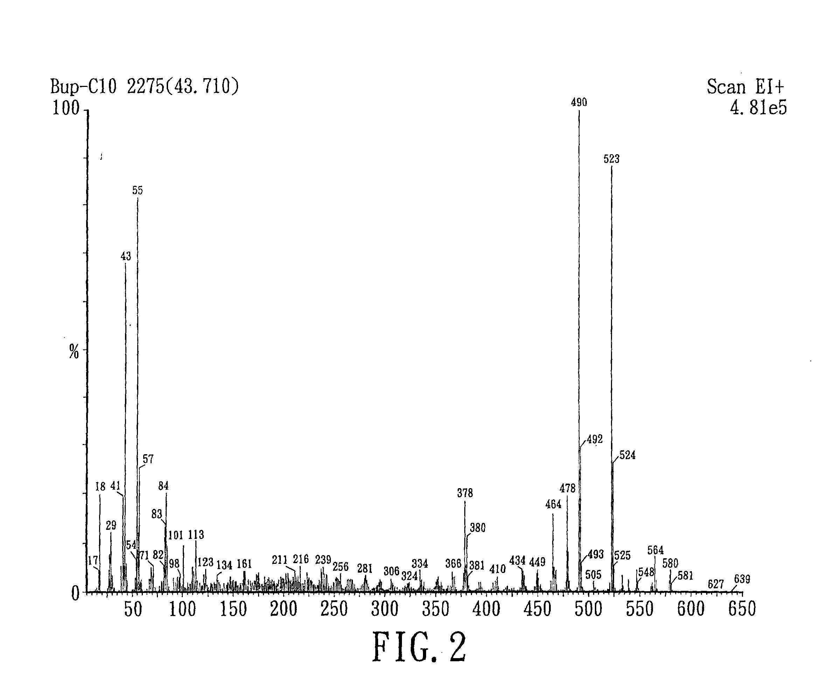

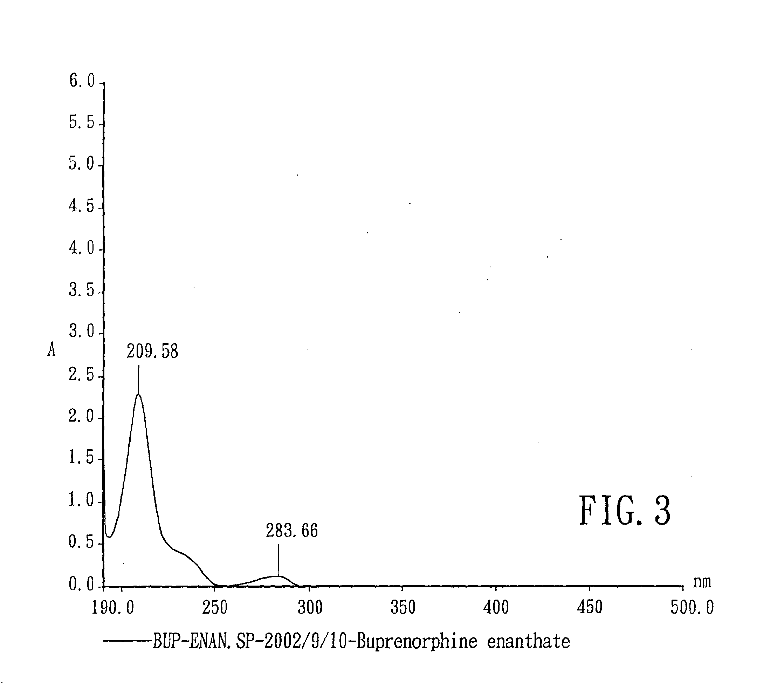

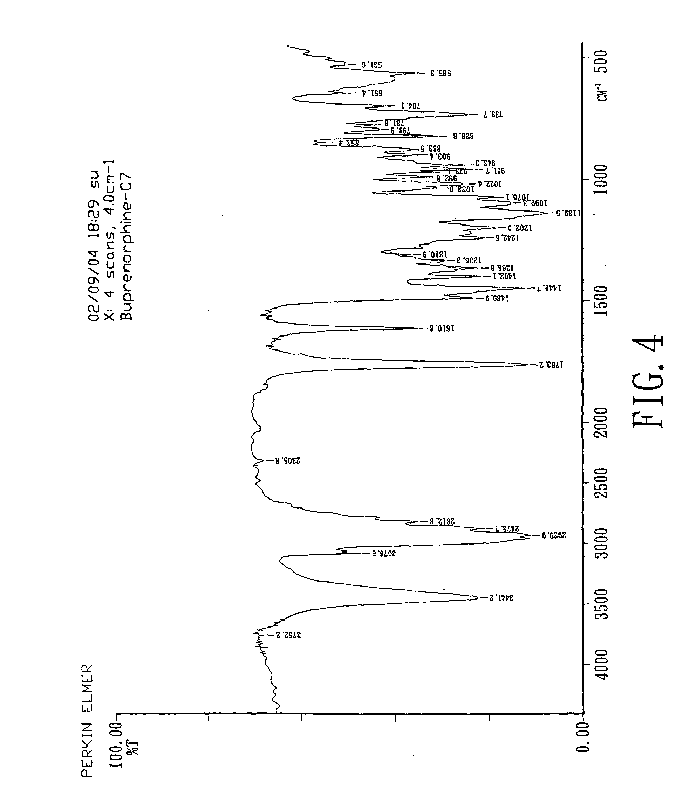

The production of the title compound was affirmed by Figures 1, 2, 3 and 4, which show the ¹H-NMR spectrum chart, the mass spectrum chart, the UV chart, and the IR chart of the title compound, respectively.
Detected properties of the title compound:
Representative ¹H-NMR (400MHz, CDCl₃) : 6.71(d,1H,J=8.1Hz), 6.52(d,1H,J=8.0Hz), 5.83(s,1H), 4.35 (s, 1H), 3.40 (s, 3H), 2.99-2.80(m, 3H), 2.59-2.43(m, 3H), 2.28-1.59(m,11H), 1.35-0.41(m.33H).
Representative mass fragments (amu) : 580, 564, 523, 490, 478, 464, 378, 113, 84, 55 (detection was carried out using GC-MS spectroscopy (Spectrum RXI, Perkin Elmer, UK)).
Representative IR absorption (cm⁻¹): 3441.2, 3076.6, 2929.9, 1763.2, 1610.8 (detection was carried out using FT-IR spectroscopy (Spectrum RXI, Perkin Elmer, UK)).

Furthermore, the physical characteristics of the title compound are shown in the following Table 2.

### Synthesis Ex. 2 Preparation of buprenorphine decanoate

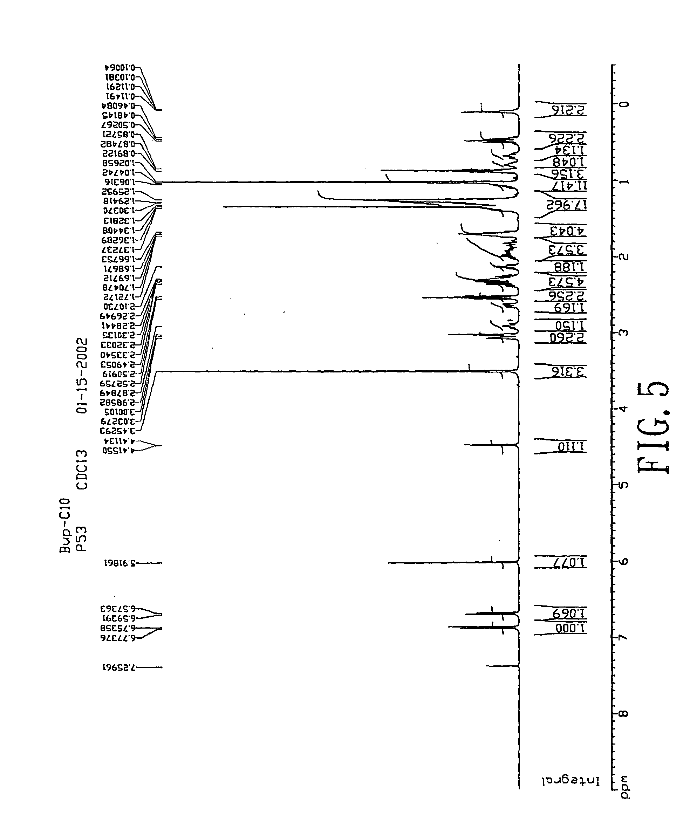

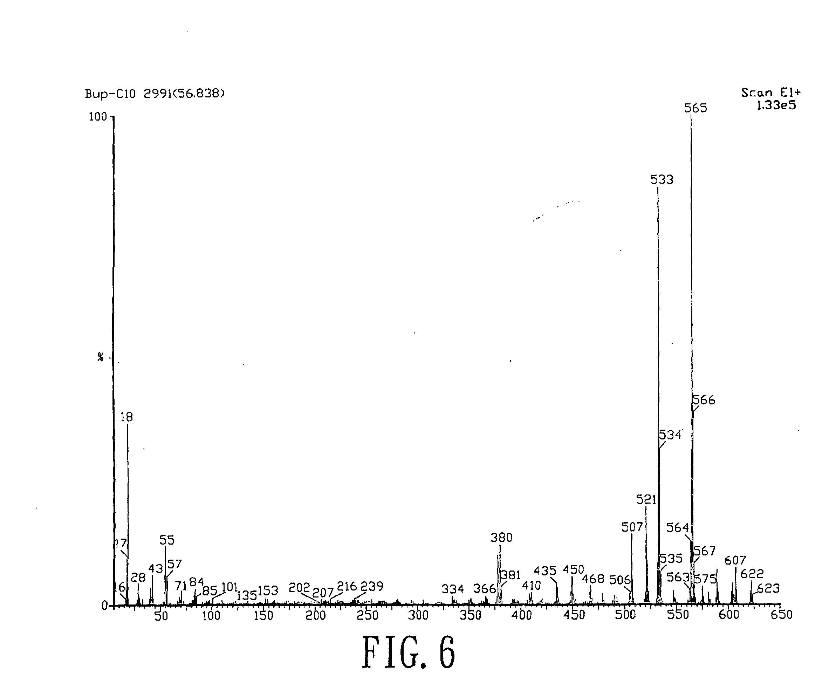

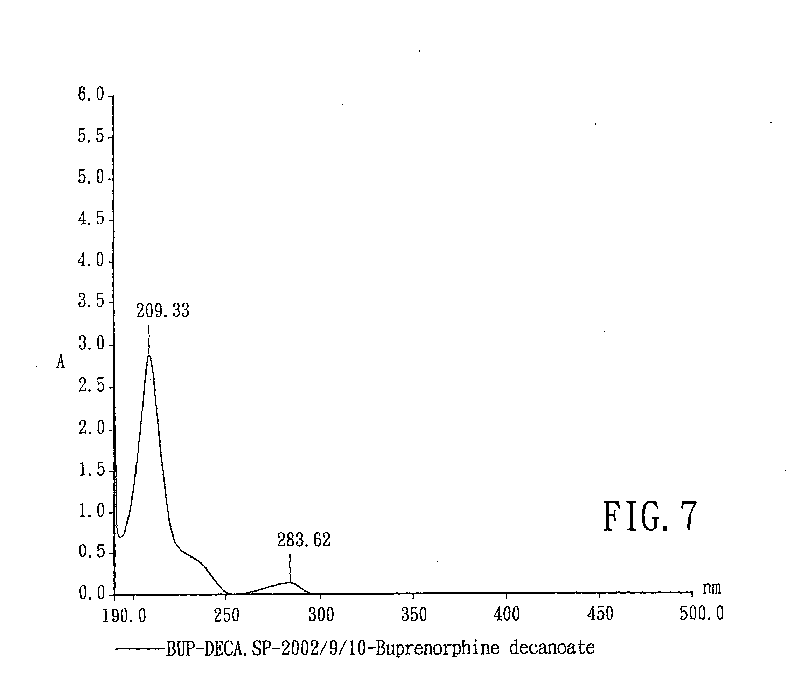

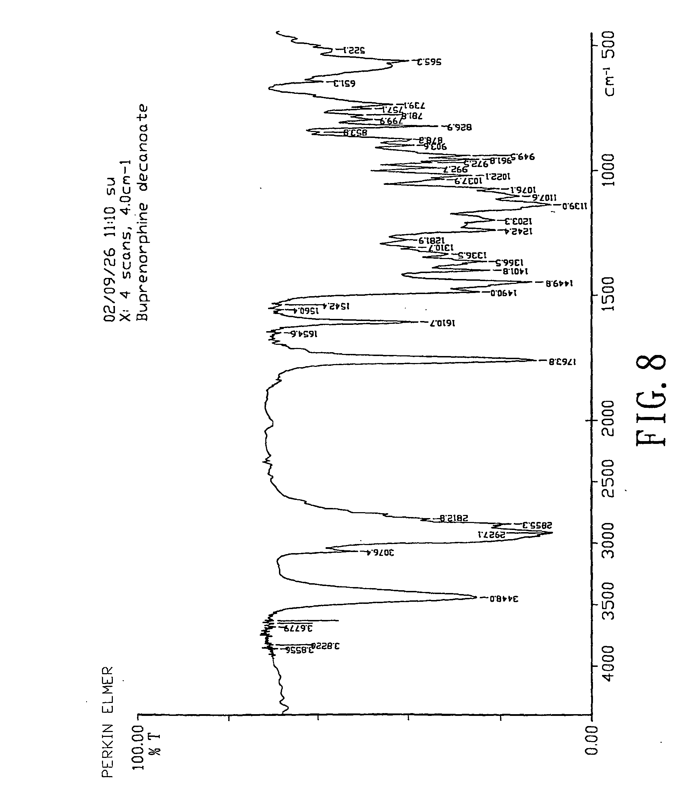

The title compound was prepared according to the procedures set forth in the above Synthesis Ex. 1, except that 0.011 mole decanoyl chloride (Fluka, Buchs, Switzerland) was used in place of heptanoyl chloride. Pure buprenorphine decanoate was obtained (see Figures 5, 6, 7 and 8, which show the ¹H-NMR spectral chart, the mass spectral chart, the UV chart, and the IR chart of the title compound, respectively).
Detected properties of the title compound:
Representative ¹H-NMR (400MHz, CDCl₃) : 6.76 (d, 1H, J=8.0Hz), 6.58(d,1H,J=8.1Hz), 5.91(s,1H), 4.41(s,1H), 3.45(s,3H), 3.00(m.2H), 2.87(m,1H), 2.50(t,2H,J=7.4Hz), 2.33-2.10(m.5H), 2.00-1.78(m,4H), 1.72-1.66(m.4H), 1.37-1.25(m,18H), 1.04(m,11H), 0.87(t,3H,J=6.6Hz), 0.80(m,1H), 0.69(m,1H), 0.48(m,2H), 0.11(m,2H).
Representative mass fragments (amu) : 622, 607, 565, 533, 521, 507, 380, 55 (detection was carried out using GC-MS spectroscopy (Spectrum RXI, Perkin Elmer, UK)).
Representative IR absorption (cm⁻¹): 3448.0, 3076.4, 2927.1, 1763.8, 1610.7 (detection was carried out using FT-IR spectroscopy (Spectrum RXI, Perkin Elmer, UK)).

Furthermore, the physical characteristics of the title compound are shown in the following Table 2.

### Synthesis Ex. 3 Preparation of buprenorphine pivalate

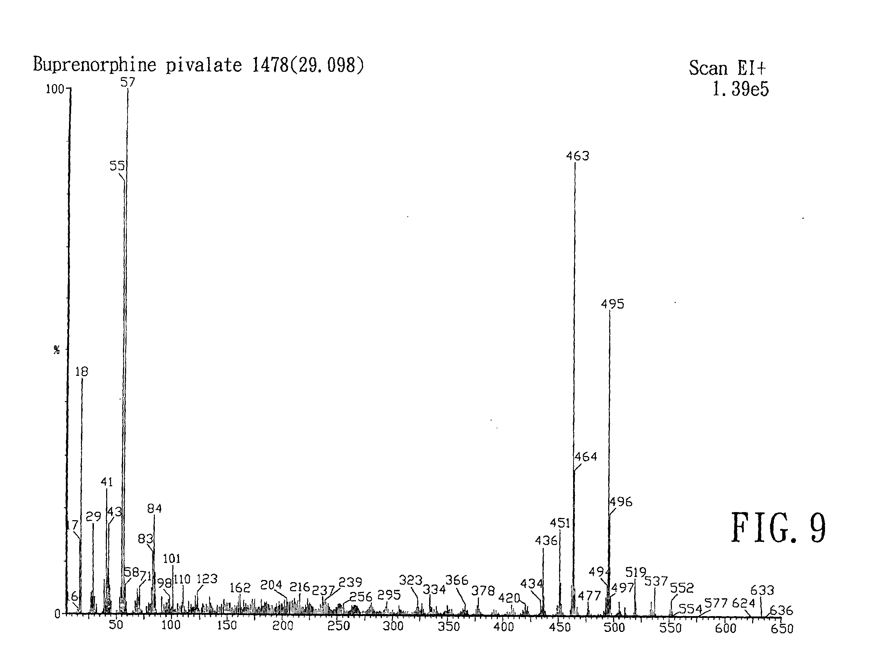

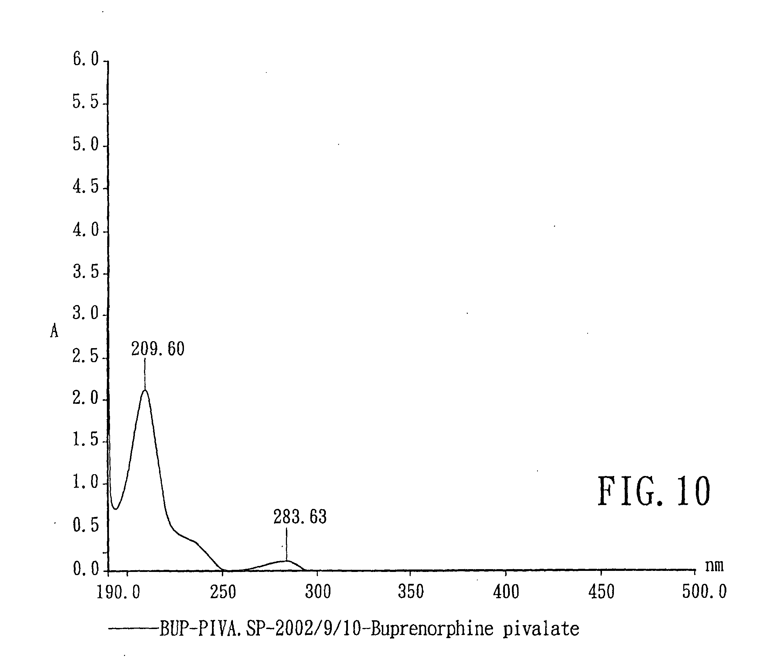

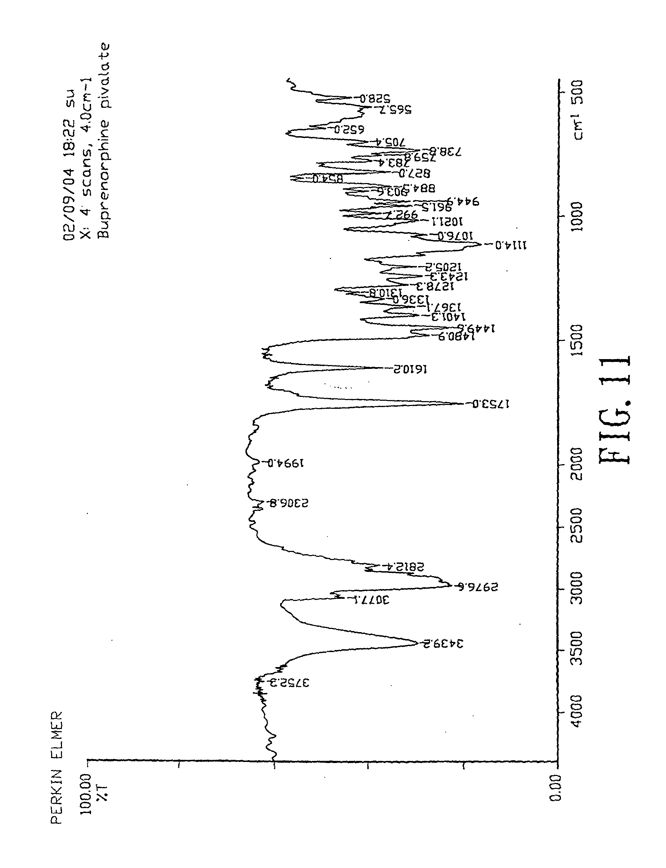

75 mL of methylene chloride and 0.01 mole of buprenorphine HCl or base were placed in a 250-mL ice-bathed round-bottomed flask. 0.011 mole of pivaloyl chloride (Acros, New Jersey, U.S.A.) dissolved in 20 mL of methylene chloride was added gradually into the flask while stirring. Following the procedures as described in the above Synthesis Ex. 1, pure buprenorphine pivalate was obtained (see Figures 9, 10 and 11, which show the mass spectral chart, the UV chart, and the IR chart of the title compound, respectively).
Detected properties of the title compound:
Representative mass fragments (amu) : 552, 537, 519, 495, 463, 451, 436, 84, 57 (detection was carried out using GC-MS spectroscopy (Spectrum RXI, Perkin Elmer, UK)).
Representative IR absorption (cm⁻¹) : 3439.2, 3077.1, 2976.6, 1753.0, 1610.2 (detection was carried out using FT-IR spectroscopy (Spectrum RXI, Perkin Elmer, UK)).

Furthermore, the physical characteristics of the title compound are shown in the following Table 2.

### Synthesis Ex. 4 Preparation of buprenorphine palmitate

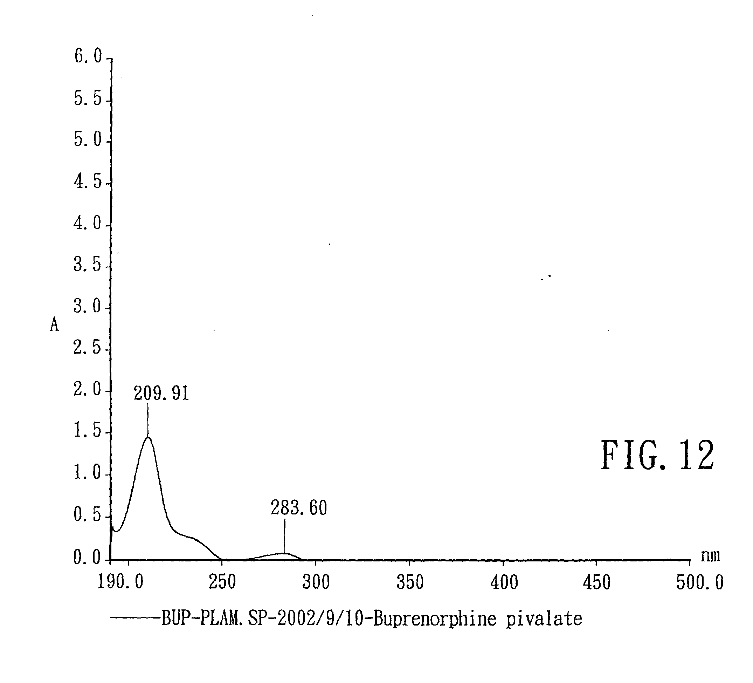

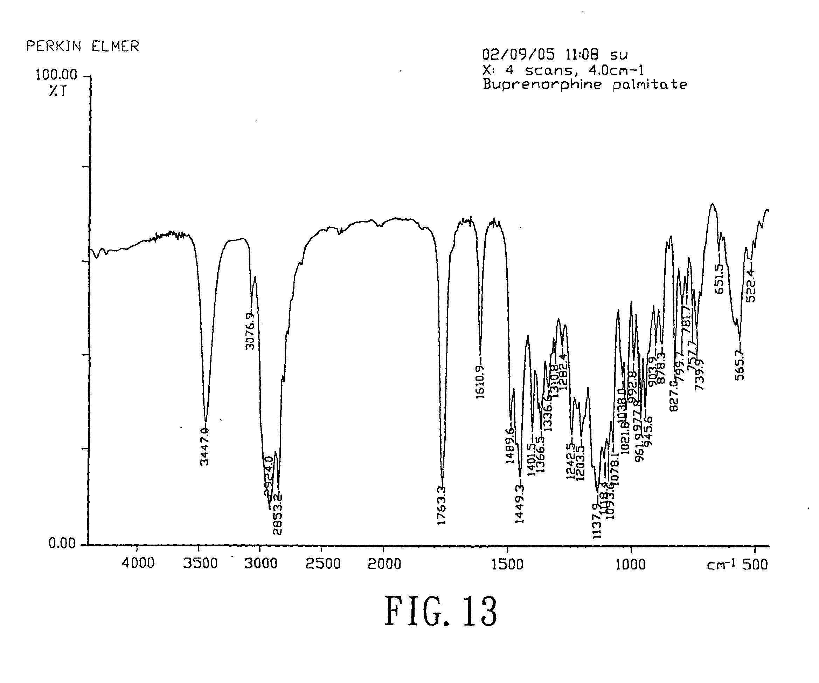

The title compound was prepared according to the procedures set forth in the above Synthesis Ex. 1, except that 0.011 mole hexadecanoyl chloride (Aldrich, Milwaukee, U.S.A.) was used in place of heptanoyl chloride. Pure buprenorphine palmitate was obtained (see Figures 12 and 13, which show the UV chart and the IR chart of the title compound, respectively).
Detected properties of the title compound:
Representative IR absorption (cm⁻¹): 3447.0, 3076.9, 2924.0, 1763.3, 1610.9 (detection was carried out using FT-IR spectroscopy (Spectrum RXI, Perkin Elmer, UK)).

Furthermore, the physical characteristics of the title compound are shown in the following Table 2.

### Synthesis Ex. 5 Preparation of dibuprenorphine pimelate

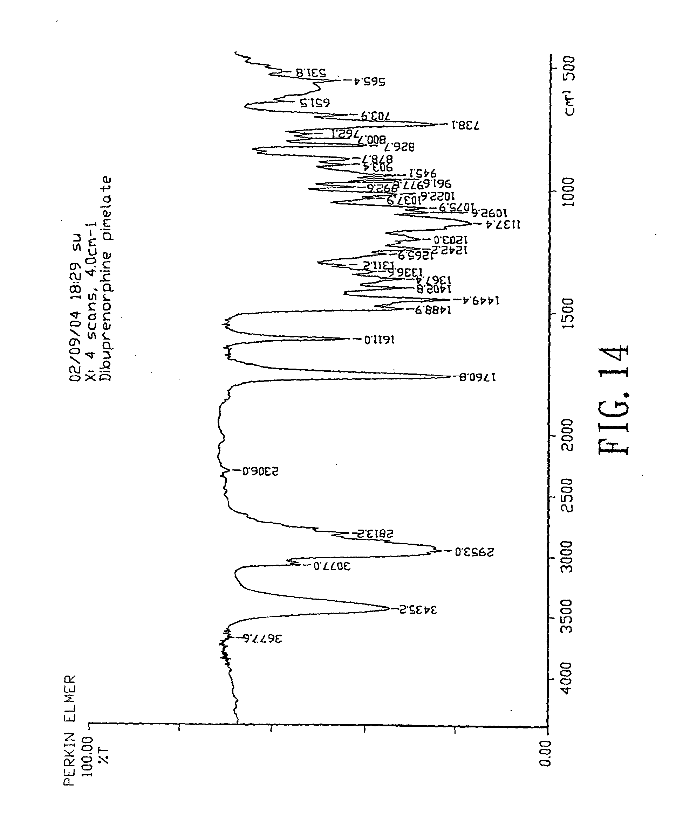

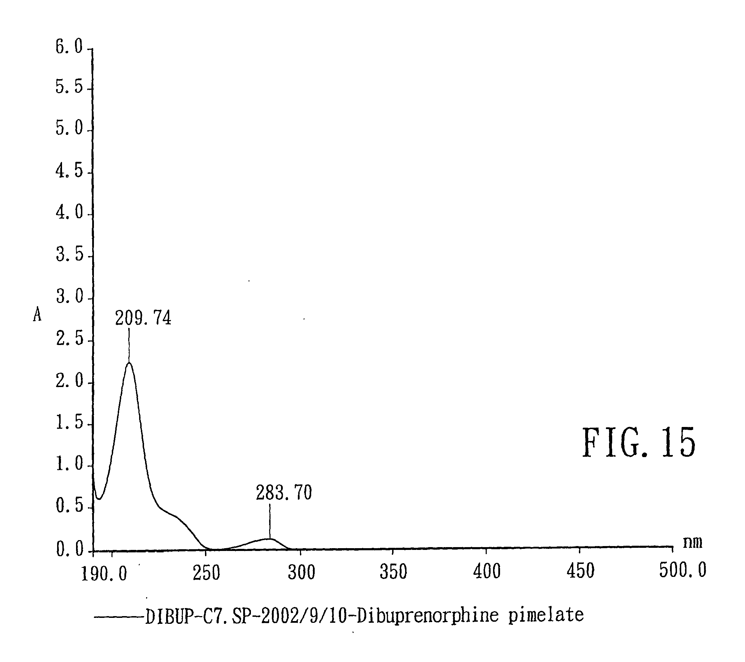

The title compound was prepared according to the procedures set forth in the above Synthesis Ex. 1, except that 0.006 mole heptanedioyl chloride (Aldrich, Milwaukee, U.S.A.) was used in place of heptanoyl chloride. Pure dibuprenorphine pimelate was obtained (see Figures 14 and 15, which show the IR chart and the UV chart of the title compound, respectively).
Detected properties of the title compound:
Representative IR absorption (cm⁻¹) : 3435.0, 3077.0, 2953.0, 1760.8, 1611.0 (detection was carried out using FT-IR spectroscopy (Spectrum RXI, Perkin Elmer, UK)).

Furthermore, the physical characteristics of the title compound are shown in the following Table 2.

### Synthesis EX. 6 Preparation of dibuprenorphine sebacoyl ester

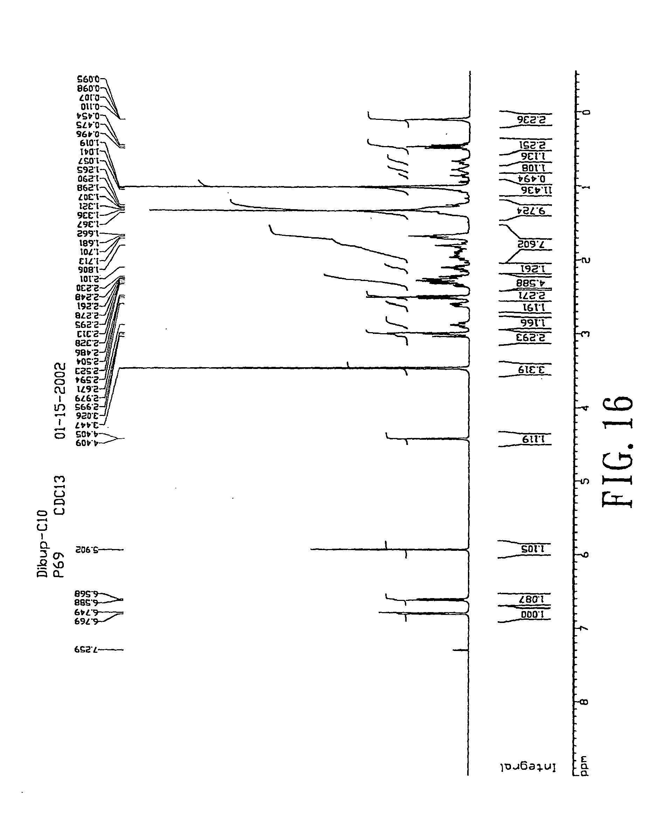

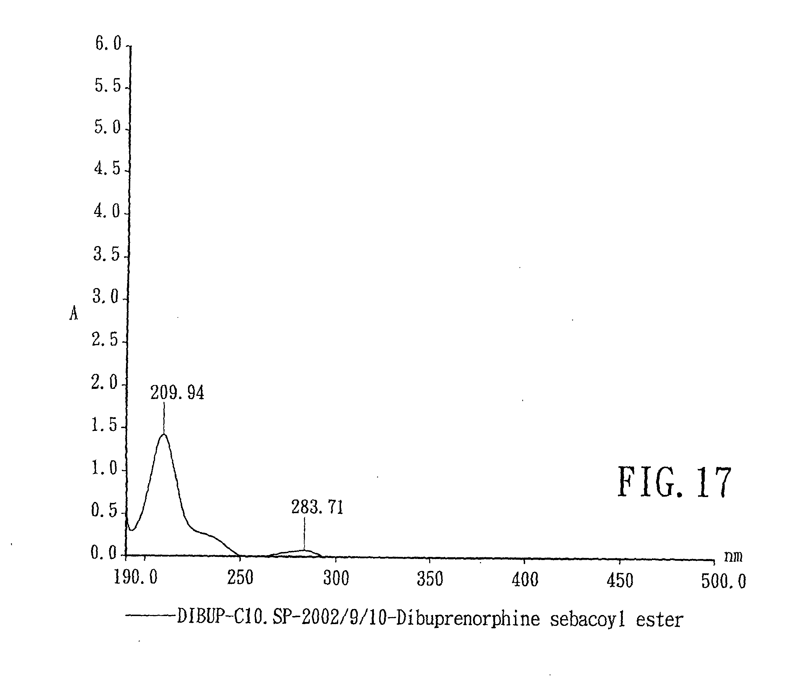

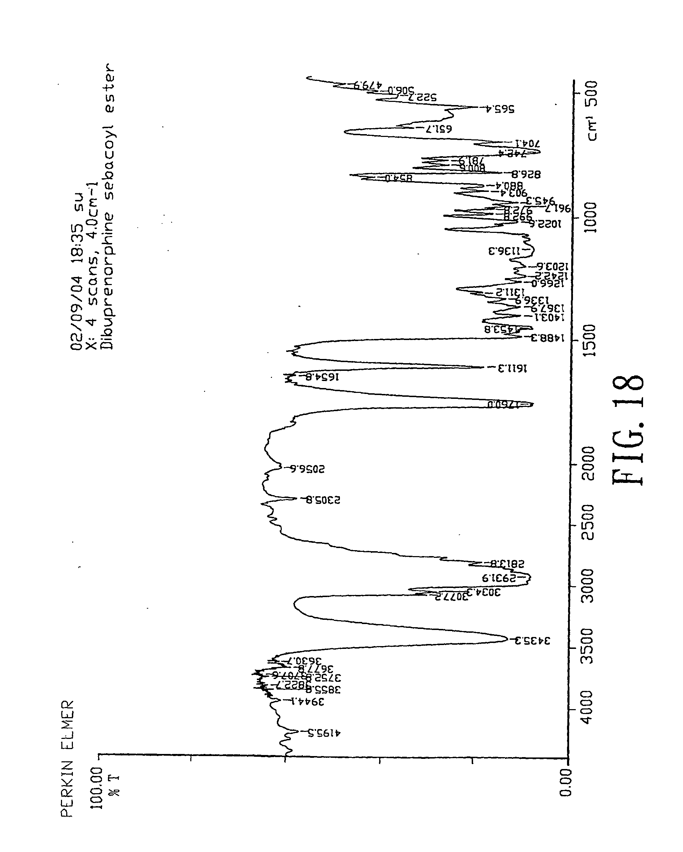

The title compound was prepared according to the procedures set forth in the above Synthesis Ex. 1, except that 0.006 mole sebacoyl chloride (Eluka, Buchs, Switzerland) was used in place of heptanoyl chloride. Pure dibuprenorphine sebacoyl ester was obtained (see Figures 16, 17 and 18, which show the ¹H-NMR spectral chart, the UV chart, and the IR chart of the title compound, respectively) .
Detected properties of the title compound:
Representative ¹H-NMR (400MHz, CDCl₃): 6.75(d,2H,J=8.0Hz), 6.57(d,2H,J=8.0Hz), 5.90(s,2H), 4.40(s,2H), 3.44(s,6H), 2.99(m,4H), 2.87(m,2H), 2.59(m,2H), 2.50(t,4H,J=7.6Hz), 2.32-2.23(m,8H), 2.10(m,2H), 2.00-1.66(m,16H), 1.36-1.26(m,18H), 1.05-1.01(m,22H), 0.80(m,2H), 0.69(m,2H), 0.47(m,4H), 0.10(m,4H).
Representative IR absorption (cm⁻¹): 3435.3, 3077.2, 2931.9, 1760.0, 1611.3 (detection was carried out using FT-IR spectroscopy (Spectrum RXI, Perkin Elmer, UK)).
Furthermore, the physical characteristics of the title compound are shown in the following Table 2.

**Table 2. The physical characteristics of buprenorphine HC1, base, and its ester derivatives**

| | | | | |
|---|---|---|---|---|
| Compound | MW | MF | MP (°C) | Ester linkage |
| Buprenorphine HCI | 504.11 | C₂₉H₄₁NO₄ • HCl | 279~281 | - |
| Buprenorphine base | 467.65 | C₂₉H₄₁NO₄ | 208~210 | - |
| Buprenorphine propionate | 523.72 | C₃₂H₄₅NO₅ | 133~135 | 1765.6 |
| Buprenorphine pivalate | 551.77 | C₃₄H₄₉NO₅ | 141~143 | 1753.0 |
| Buprenorphine benzoate | 571.76 | C₃₆H₄₅NO₅ | 168~170 | 1742.1 |
| Buprenorphine enanthate | 579.83 | C₃₆H₅₃NO₅ | 84~86 | 1763.2 |
| Buprenorphine decanoate | 621.91 | C₃₉H₅₉NO₅ | 87~89 | 1763.8 |
| Buprenorphine palmitate | 706.07 | C₄₅H₇₁NO₅ | < 0 | 1763.3 |
| Dibuprenorphine pimelate | 1059.45 | C₆₅H₉₀N₂O₁₀ | 103~105 | 1760.8 |
| Dibuprenorphine sebacoyl ester | 1101.53 | C₆₈H₉₆N₂O₁₀ | 89~90 | 1760.0 |

| | | | | |
|---|---|---|---|---|
| Infra-red spectrum of each compound was detected using FT-IR spectroscopy (Spectrum RXI, Perkin Elmer, UK) | | | | |

### Preparation Ex. 1 Preparation of injectable oil formulations containing buprenorphine base or buprenorphine ester derivatives of this invention

(1) 10 *µ* Mole of buprenorphine base was added into 1 mL of sesame oil. The mixture was shaken slightly to effect complete dissolution.
(2) 20 *µ*Mole of buprenorphine propionate was added into 1 mL of sesame oil. The mixture was shaken slightly to effect complete dissolution.
(3) 20 *µ*Mole of buprenorphine decanoate was added into 1 mL of sesame oil. The mixture was shaken slightly to effect complete dissolution.
(4) 20 µMole of buprenorphine pimelate was added into 1 mL of sesame oil. The mixture was shaken slightly to effect complete dissolution.
(5) 20 *µ*Mole of buprenorphine ester or polyester was added into 1 mL of ethyl ester of peanut oil or soybean oil. The mixture was shaken slightly to effect complete dissolution.

### Pharmacological Example 1

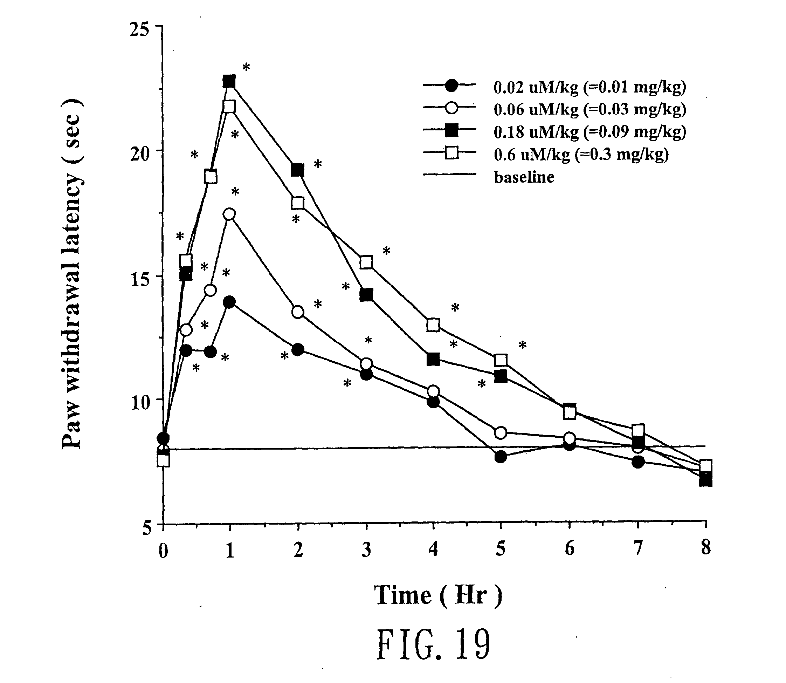

*In vivo* analgesic efficacy of buprenorphine hydrochloride via intramuscular injection(dose-finding study)
(1) Animal: male Sprague-Dawley rats (175-225 gm, 6 weeks old), n=6 in each group.
(2) Drugs : buprenorphine hydrochloride solution in 0.9% saline, 0.02 µMole/kg (=0.01 mg/kg), 0.06µ Mole/kg (=0.03 mg/kg), 0.18 µmole /kg (=0.09 mg/kg), 0.6 µMole/kg (=0.3 mg/kg), intramuscular injection in a tested rat's right hind leg.
(3) Test: plantar test, using the device (7370, UGO, BASILE, Italy).
   The plantar test enables the researcher to discern a peripherally mediated response to thermal stimulation caused by drugs in the unrestrained rat. It basically consists of a movable I.R. (infrared) Generator placed below a glass pane upon which the operator deposits the rat. A Perspex enclosure defines the space within which the animal is unrestrained. It is divided into three compartments, which help the operator to carry out a rapid "screening" work: up to three rats can be tested with no appreciable delay therebetween.
   The operator positions the I.R. Generator directly beneath the hind paw of the rat and activates via a START key both the I.R. Source and a reaction time counter. When the rat feels pain and withdraws its paw, the I .R. Generator is automatically switched off and the timer stops, determining the withdrawal latency.
   Concerning the detail of the plantar test, reference may be made to, e.g., K.M. Hargreaves et al., "A New and Sensitive Method for Measuring Thermal Nociception in Cutaneous Hyperalgesia," Pain 32:77-88, 1988, and K. M. Hargreaves et al., "Peripheral Action of Opiates in the Blockade of Carrageenan-Induced Inflammation" Pain Research and Clinical Management, Vol. 3. Elsevier Science Publishers, Amsterdam: 55-60, 1988.
   The latency from the time of stimulus (radiant heat) to paw (left hind leg) withdrawal was assigned as response latency. Radiant heat was set to provide a predrug latency of 7-9 seconds. To prevent tissue damage, a 25-second cut-off time was set.
(4) Statistics: Data are shown as mean. "*" means P<0. 05 when compared to pretest value using ANOVA and Dunnett test. ANOVA is analysis of variance frequently, abbreviated as ANOVA, and is an extremely powerful statistical technique which can be used to separate and estimate the different causes of variation. Dunnett test is a posterior comparison among means following an ANOVA. This test allows one to make all possible comparisons among groups. P means probability, in which P<0.05 means significantly different in statistics.
(5) Results: Buprenorphine HCI of different doses demonstrated a duration of analgesic action of 3-5 hr (see Table 3 and Figure 19).

### Pharmacological Example 2

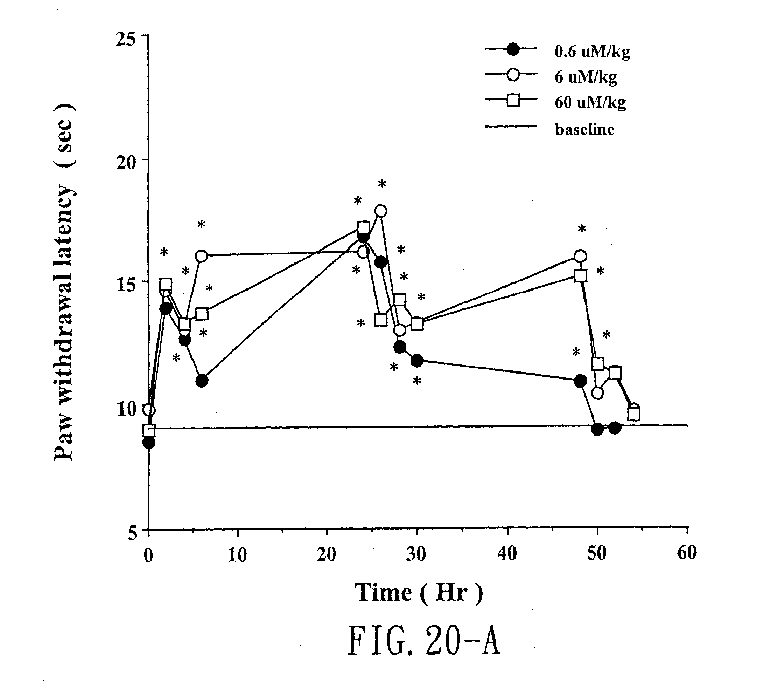

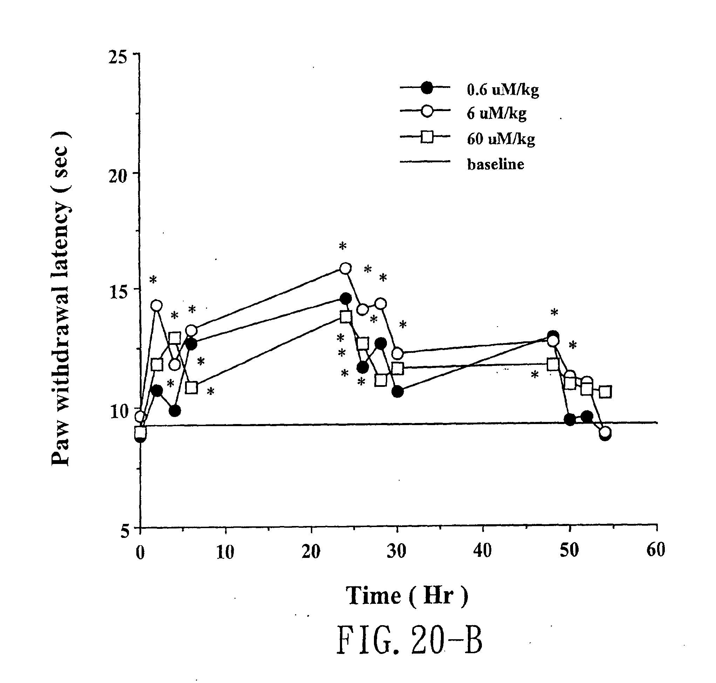

*In vivo* dose-finding studies of buprenorphine base via either intramuscular or subcutaneous injection
(1) Animal: male Sprague-Dawley rats (175-225 gm) (n=6)
(2) drugs: buprenorphine base in sesame oil, 0.6 µM/kg, 6µM/kg, 60 µM/kg; intramuscular injection in a tested rat's right hind leg.
(3) Test: plantar test (see Pharmacological Example 1 described above).
(4) Statistics: ANOVA followed by Dunnett test. Data are shown as "means."
(5) Results: Buprenorphine base of different doses via intramuscular injection demonstrated a duration of analgesic action of 48-50 hours (see Table 3 and Figure 20-A) . Likewise, buprenorphine base of different doses via subcutaneous injection demonstrated a duration of action of 48-50 hours (see Table 3 and Figure 20-B).

### Pharmacological Example 3

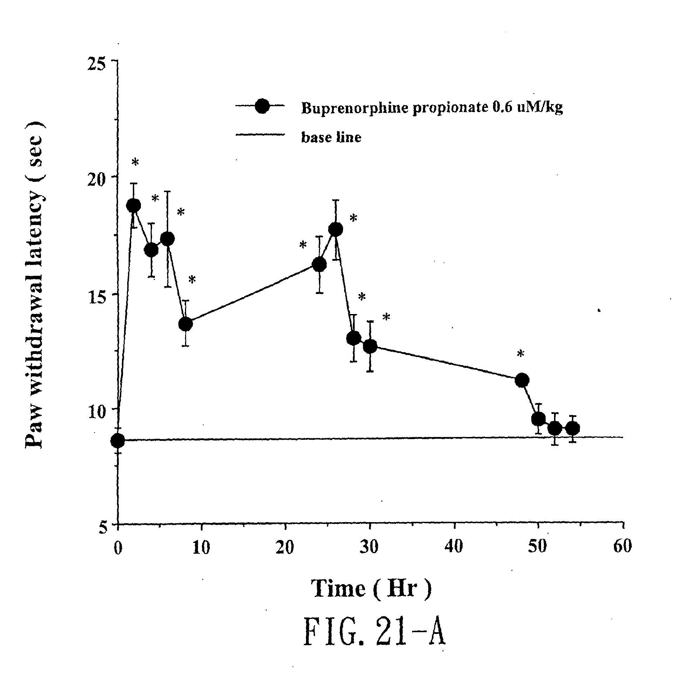

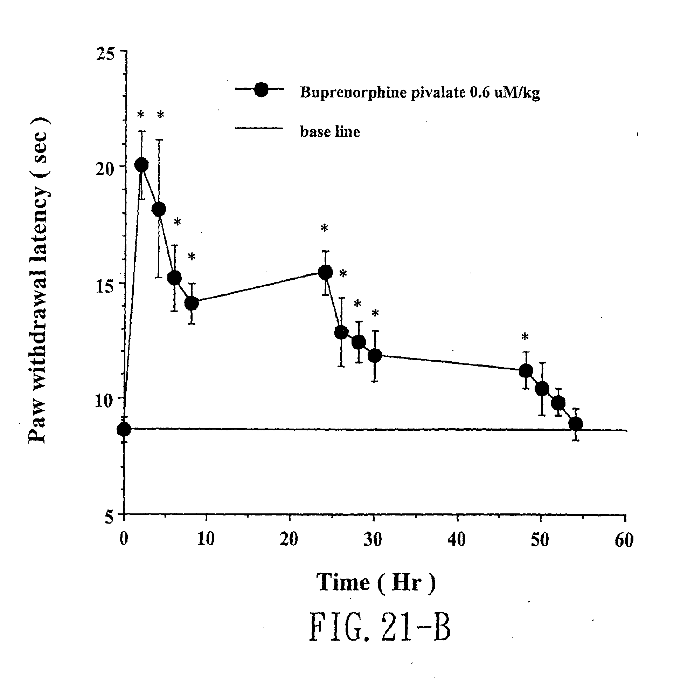

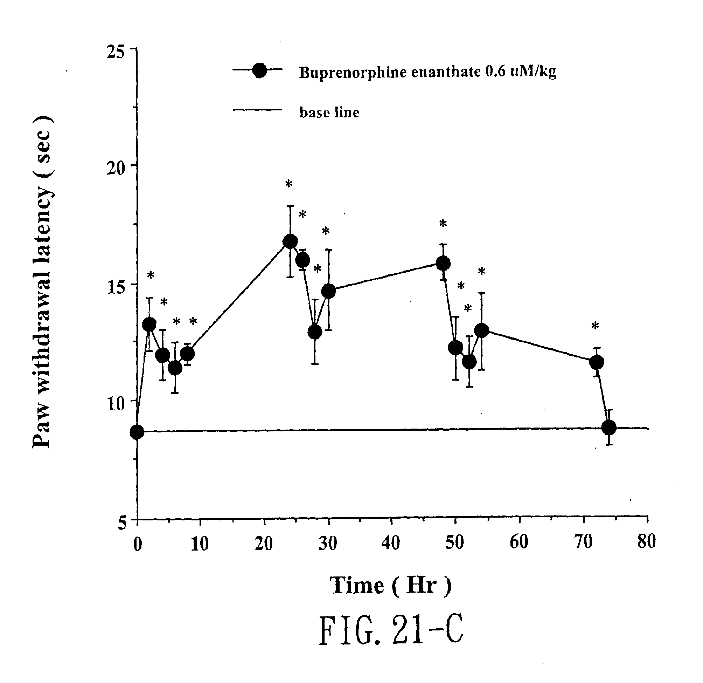

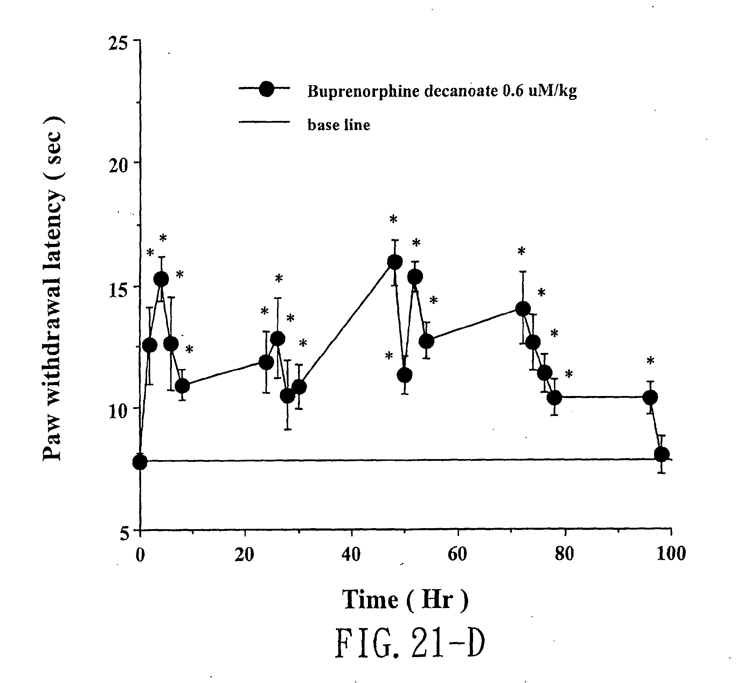

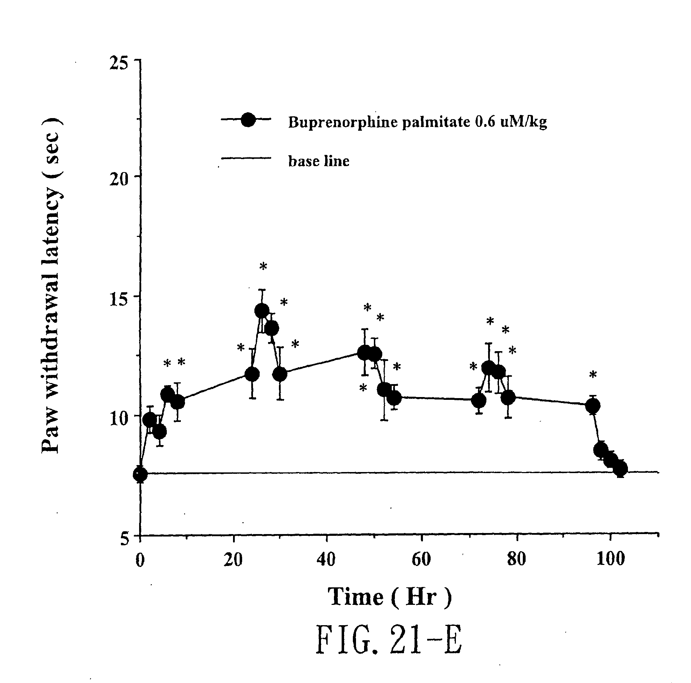

In vivo analgesic efficacies of five buprenorphine monocarboxylic ester derivatives of formula (I) via intramuscular injection
(1) Animal: male Sprague-Dawley rats (175-225 gm), n = 6 in each group.
(2) drugs: buprenorphine propionate 0.6 *µ* M/kg, buprenorphine pivalate 0.6 *µ* M/kg, buprenorphine enanthate 0.6 *µ* M/kg, buprenorphine decanoate 0.6 *µ* M/kg, and buprenorphine palmitate 0.6 *µ*M/kg. All of these ester derivatives were dissolved in sesame oil as an oil solution and injected intramuscularly in the right hind leg.
(3) Test: plantar test (see Pharmacological Example 1 described above).
(4) Statistics: ANOVA followed by Dunnett test (see Pharmacological Example 1 described above).
(5) Results: Intramuscular injection of the tested five buprenorphine monocarboxylic ester derivatives of this invention at a dose of 0.6 µM/kg demonstrated a rapid onset of action with a duration of 48-96 hours, and in particular, both buprenorphine decanoate and palmitate could provide a duration of action of 96 hours (see Table 3 and Figures 21-A to 21-E).

### Pharmacological Example 4

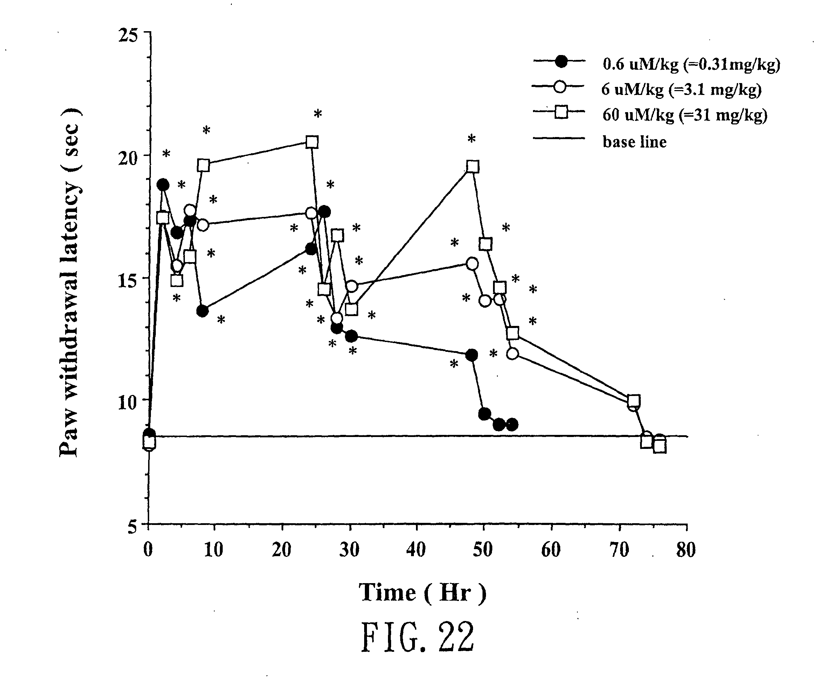

In vivo dose-finding studies of buprenorphine propionate via intramuscular injection
(1) Animal: male Sprague-Dawley rats (175-225 gm), n = 6 in each group.
(2) drugs: buprenorphine propionate (oil solution), dose: 0.6 µ M/kg, 6 µ M/kg, 60 µ M/kg;intramuscular injection in a tested rat's right hind leg.
(3) Test: plantar test (see Pharmacological Example 1 described above).
(4) Statistics: ANOVA followed by Dunnett test (see Pharmacological Example 1 described above).
(5) Results: Buprenorphine propionate of different doses via intramuscular injection demonstrated a duration of action of 48-60 hours (see Table 3 and Figure 22).

### Pharmacological Example 5

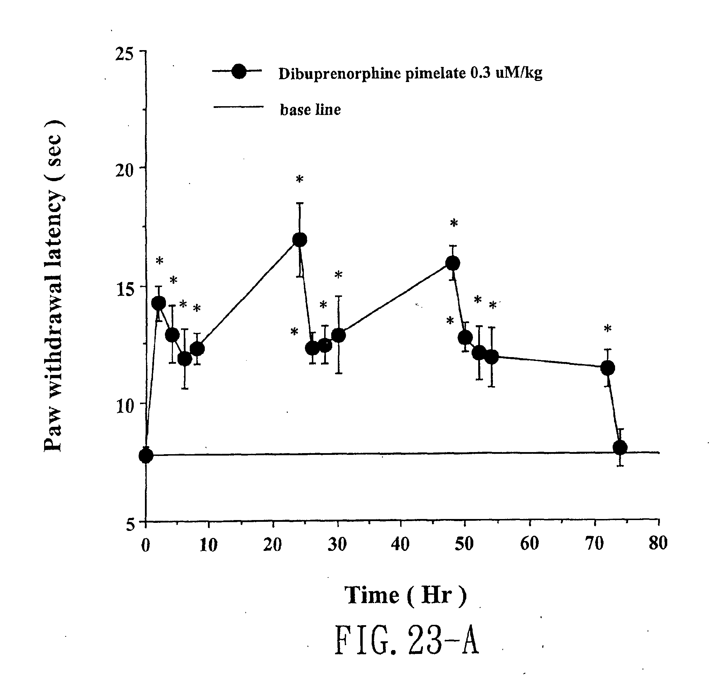

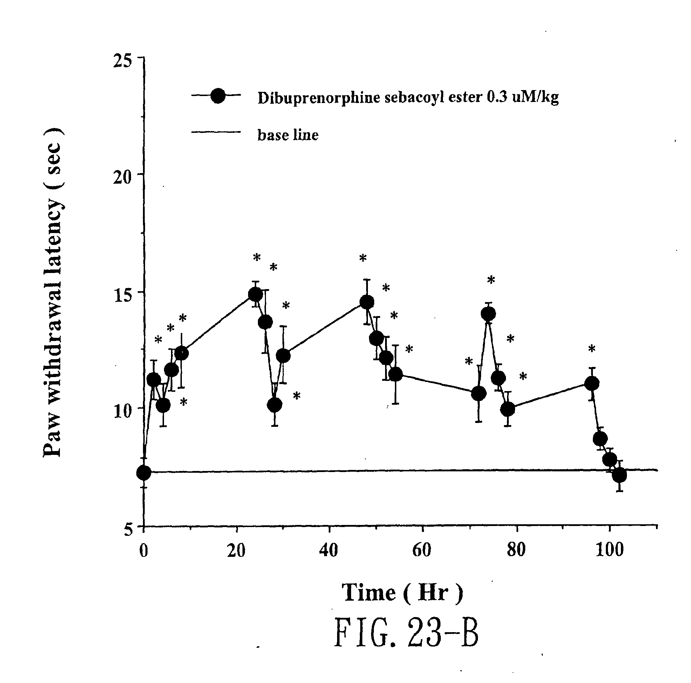

*In vivo* analgesic efficacies of two dibuprenorphine dicarboxylic ester derivatives of formula (II) via intramuscular injection
(1) Animal: male Sprague-Dawley rats (175-225 gm), n = 6 in each group.
(2) drugs: Dibuprenorphine pimelate, dibuprenorphine sebacoyl ester, dose: 0.3 µM/kg (oil solution in sesame oil), intramuscular injection in a tested rat's right hind leg.
(3) Test: plantar test (see Pharmacological Example 1 described above).
(4) Statistics: ANOVA followed by Dunnett test (see Pharmacological Example 1 described above).
(5) Results: Both Buprenorphine pimelate and sebacoyl ester at a dose of 0.3 *µ* M/kg via intramuscular injection demonstrated a rapid onset of action (2 hours) with duration of action of 72 and 96 hours (see Table 3 and Figures 23-A and 23-B).

The following Table 3 summarizes the analgesic duration of buprenorphine and its derivatives in rats using the plantar test.

**Table 3. The analgesic durations of buprenorphine and its derivatives in rats using the plantar test**

| Compound | Dose (µM/kg) | Route of administratio | Analgesic duration (h) |
|---|---|---|---|
| Buprenorphine HCl | 0.6 | IM | 5 |
| Buprenorphine base | 0.6; 6; 60 | IM | 48; 48; 50 |
| Buprenorphine base | 0.6; 6; 60 | SC | 48; 50; 48 |
| Buprenorphine propionate | 0.6; 6; 60 | IM | 48; 54; 54 |
| Buprenorphine pivalate | 0.6 | IM | 48 |
| Buprenorphine benzoate | 0.6 | IM | 72 |
| Buprenorphine enanthate | 0.6 | IM | 72 |
| Buprenorphine decanoate | 0.6 | IM | 96 |
| Buprenorphine palmitate | 0.6 | IM | 96 |
| Dibuprenorphine pimelate | 0.3 | IM | 72 |
| Dibuprenorphine sebacoyl ester | 0.3 | IM | 96 |

| | | | |
|---|---|---|---|
| IM: intramuscular, SC: subcutaneous | | | |

## Claims

1. A dibuprenorphine dicarboxylic ester derivative of formula (II): 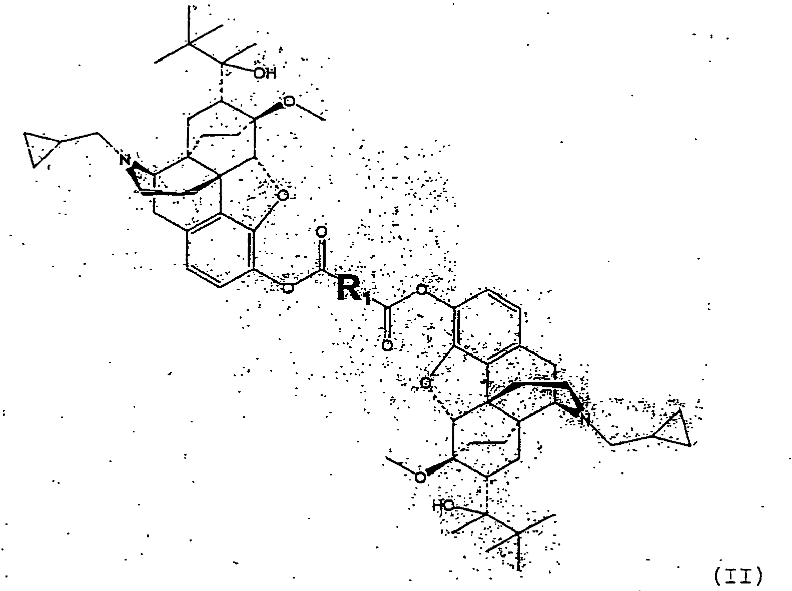 wherein R₁ is a divalent moiety of a saturated or unsaturated aliphatic group having 1 to 40 carbon atoms and optionally substituted with a phenyl group.

2. A dibuprenorphine dicarboxylic ester derivative as claimed in claim 1, wherein R₁ is an alkylene group having 1 to 40 carbon atoms.

3. A dibuprenorphine dicarboxylic ester derivative as claimed in claim 2, wherein R₁ is an alkylene group having 1 to 20 carbon atoms.

4. A dibuprenorphine dicarboxylic ester derivative as claimed in claim 1, which is dibuprenorphine pimelate or dibuprenorphine sebacoyl ester.

5. An analgesic pharmaceutical composition comprising:
(a) a therapeutically effective amount of:
(i) a buprenorphine monocarboxylic ester derivative of formula (I): 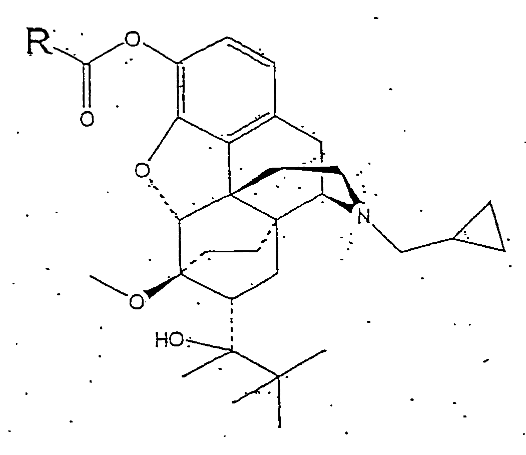 wherein R is selected from a straight-chain or branched saturated or unsaturated aliphatic group optionally substituted with an aryl group, or an aryl group optionally substituted with a straight-chain or branched saturated or unsaturated aliphatic group;
with the proviso that R is not selected from methyl, ethyl, propyl, n-butyl, n-pentyl, n-hexyl or isopropyl; or
(ii) a dibuprenorphine dicarboxylic ester derivative as claimed in any of claims 1 to 4; and
(b) a pharmaceutically acceptable oil carrier.

6. An analgesic pharmaceutical composition as claimed in claim 5, wherein said carrier is sesame oil castor oil, cotton seed oil, soybean oil, peanut oil or ethyl ester of peanut oil, or a combination thereof.

7. An analgesic pharmaceutical composition as claimed in claim 5 or claim 6, comprising a buprenorphine monoester derivative of Formula (I), wherein R is an alkyl group optionally substituted with a phenyl group.

8. An analgesic pharmaceutical composition as claimed in claim 5 or claim 6, comprising a buprenorphine monocarboxylic ester derivative of Formula (I), wherein R is an alkyl group having 2 to 40 carbon atoms.

9. An analgesic pharmaceutical composition as claimed in claim 5 or claim 6, comprising a buprenorphine monocarboxylic ester derivative of Formula (I), wherein R is an alkyl group having 5 to 20 carbon atoms.

10. An analgesic pharmaceutical composition as claimed in claim 5 or claim 6, comprising a buprenorphine monocarboxylic ester derivative of Formula (I), wherein R is selected from a straight-chain alkyl group optionally substituted with a phenyl group, a branched alkyl group optionally substituted with a phenyl group, a phenyl group optionally substituted with a straight-chain aliphatic group, or a phenyl group optionally substituted with a branched aliphatic group.

11. An analgesic pharmaceutical composition as claimed in claim 5 or claim 6, comprising a buprenorphine monocarboxylic ester derivative of Formula 1, which is buprenorphine pivalate, buprenorphine benzoate, buprenorphine decanoate or buprenorphine palmitate.

12. Use of a composition comprising a therapeutically effective amount of buprenorphine base and an injectable oil and substantially free of water in the manufacture of an injectable medicament for providing a prolonged analgesia to a human or animal.

13. Use of a composition as claimed in claim 12, wherein said injectable oil vehicle is sesame oil, castor oil, cotton seed oil, soybean oil, peanut oil or ethyl ester of peanut oil, or a combination thereof.

14. An analgesic pharmaceutical composition as claimed in any of claims 5 to 11, which is for intramuscular or subcutaneous administration.

15. The use of a dibuprenorphine dicarboxylic ester derivative as claimed in any of claims 1 to 4 for the preparation of an injectable medicament for providing prolonged analgesia to an animal or human.

16. The use of a combination of (i) a buprenorphine monocarboxylic ester derivative as defined in part (i) of claim 5 or a dibuprenorphine dicarboxylic ester derivative as claimed in any of claims 1 to 4; and (ii) a pharmaceutically acceptable oil vehicle, for the preparation of a medicament for providing prolonged analgesia to an animal or human.

17. A method of preparing a dibuprenorphine dicarboxylic ester derivative of formula (II) as claimed in claim 1, comprising the steps of:
(i) treating buprenorphine HCl or base with trimethylamine in the presence of methylene chloride; and
(ii') adding to the mixture from step (i') a compound of formula R₁ (COOH)₂, or an acid anhydride or an acid chloride thereof, in the presence of methylene chloride, wherein R₁ in the formula R₁ (COOH)₂ is a divalent moiety of a saturated or unsaturated aliphatic group having 1 to 40 carbon atoms and optionally substituted with a phenyl group.

18. A method as claimed in claim 17, wherein the compound of R₁ (COOH)₂ used in step (ii') is an aliphatic dicarboxylic acid having 30-40 carbon atoms, or an acid anhydride or an acid chloride thereof.

19. A method as claimed in claim 17, wherein heptanedioyl chloride or sebacoyl acid is used in step (ii').

## Patentansprüche

1. Ein Dibuprenorphin-Dicarbonsäureester-Derivat mit Formel (II): 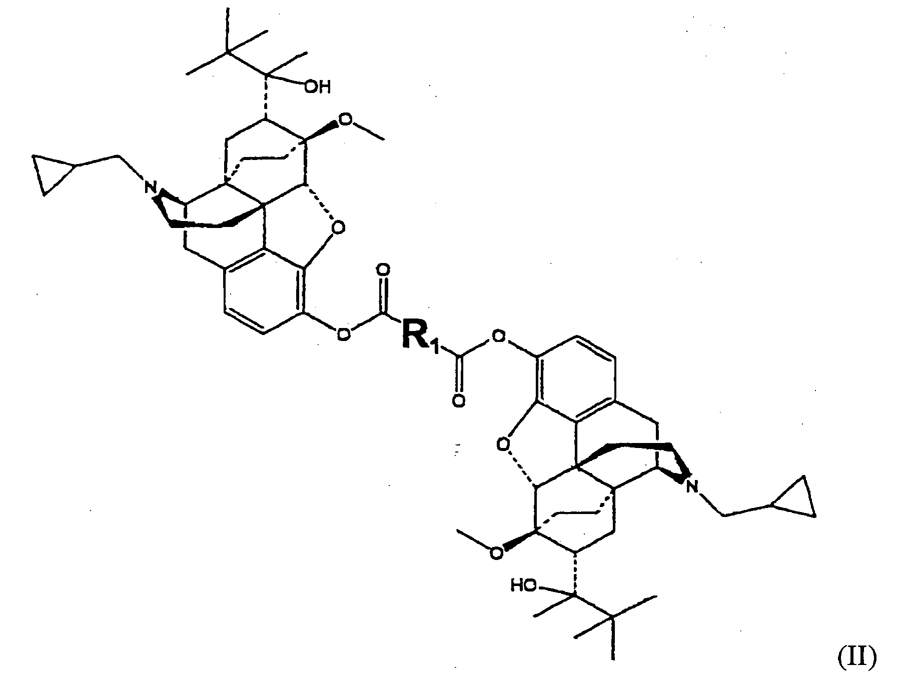 wobei R₁ eine bivalente Komponente einer gesättigten oder ungesättigten aliphatischen Gruppe mit 1 bis 40 Kohlenstoffatomen ist, die optional mit einer Phenylgruppe substituiert ist.

2. Ein Dibuprenorphin-Dicarbonsäureester-Derivat nach Anspruch 1, wobei R₁ eine Alkylengruppe mit 1 bis 40 Kohlenstoffatomen ist.

3. Ein Dibuprenorphin-Dicarbonsäureester-Derivat nach Anspruch 2, wobei R₁ eine Alkylengruppe mit 1 bis 20 Kohleristoffatomen ist.

4. Ein Dibuprenorphin-Dicarbonsäureester-Derivat nach Anspruch 1, das Dibuprenorphin-Pimelat oder Dibuprenorphin-Sebacoylester ist.

5. Eine analgetische pharmazeutische Zusammensetzung, enthaltend:
(a) eine therapeutisch wirksame Menge von:
(i) einem Buprenorphin-Monocarbonsäureester-Derivat mit Formel (I) 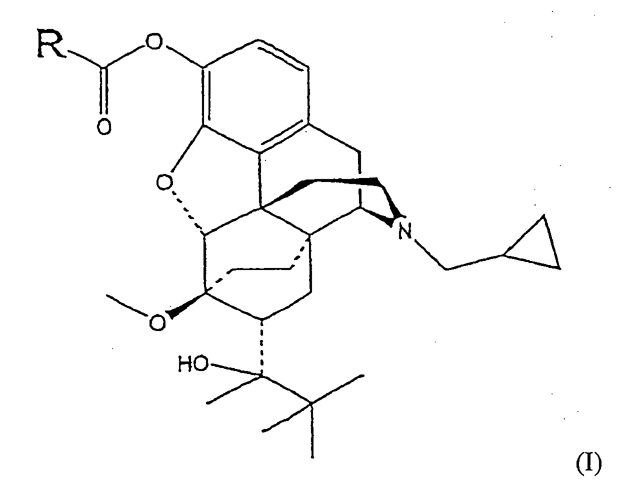 wobei R ausgewählt ist aus einer geradkettigen oder verzweigten, gesättigten oder ungesättigten aliphatischen Gruppe, die optional mit einer Arylgruppe substituiert ist, oder einer Arylgruppe, die optional mit einer geradkettigen oder verzweigten, gesättigten oder ungesättigten aliphatischen Gruppe substituiert ist;
mit der Maßgabe, dass R nicht ausgewählt ist aus Methyl, Ethyl, Propyl, n-Butyl, n-Pentyl, n-Hexyl oder Isopropyl; oder
(ii) einem Dibuprenorphin-Dicarbonsäureester-Derivat nach einem der Ansprüche 1 bis 4; und
(b) eine pharmazeutisch verträgliche Ölträgersubstanz.

6. Eine analgetische pharmazeutische Zusammensetzung nach Anspruch 5, wobei die Trägersubstanz Sesamöl, Rizinusöl, Bauwollsamenöl, Sojabohnenöl, Erdnussöl oder Ethylester von Erdnussöl, oder eine Kombination davon ist.

7. Eine analgetische pharmazeutische Zusammensetzung nach Anspruch 5 oder Anspruch 6, die ein Buprenorphin-Monoester-Derivat mit Formel (I) enthält, wobei R eine Alkylgruppe ist, die optional mit einer Phenylgruppe substituiert ist.

8. Eine analgetische pharmazeutische Zusammensetzung nach Anspruch 5 oder Anspruch 6, die ein Buprenorphin-Monocarbonsäureester-Derivat mit Formel (I) enthält, wobei R eine Alkylgruppe mit 2 bis 40 Kohlenstoffatomen ist.

9. Eine analgetische pharmazeutische Zusammensetzung nach Anspruch 5 oder Anspruch 6, die ein Buprenorphin-Monocarbonsäureester-Derivat mit Formel (I) enthält, wobei R eine Alkylgruppe mit 5 bis 20 Kohlenstoffatomen ist.

10. Eine analgetische pharmazeutische Zusammensetzung nach Anspruch 5 oder Anspruch 6, die ein Buprenorphin-Monocarbonsäureester-Derivat mit Formel (I) enthält, wobei R ausgewählt ist aus einer geradkettigen Alkylgruppe, die optional mit einer Phenylgruppe substituiert ist, einer verzweigten Alkylgruppe, die optional mit einer Phenylgruppe substituiert ist, einer Phenylgruppe, die optional mit einer geradkettigen aliphatischen Gruppe substituiert ist, oder einer Phenylgruppe, die optional mit einer verzweigten aliphatischen Gruppe substituiert ist.

11. Eine analgetische pharmazeutische Zusammensetzung nach Anspruch 5 oder Anspruch 6, die ein Buprenorphin-Monocarbonsäureester-Derivat mit Formel (I) enthält, die Buprenorphin-Pivalat, Buprenorphin-Benzoat, Buprenorphin-Decanoat oder Buprenorphin-Palmitat ist.

12. Verwendung einer Zusammensetzung, die eine therapeutisch wirksame Menge einer Buprenorphin-Base und ein injizierbares Öl enthält, und im Wesentlichen wasserfrei ist, zur Herstellung eines injizierbaren Medikaments zum Bereitstellen einer verlängerten Analgesie bei einem Mensch oder Tier.

13. Verwendung einer Zusammensetzung nach Anspruch 12, wobei das injizierbare Ölvehikel Sesamöl, Rizinusöl, Bauwollsamenöl, Sojabohnenöl, Erdnussöl oder Ethylester von Erdnussöl, oder eine Kombination davon ist.

14. Eine analgetische pharmazeutische Zusammensetzung nach einem der Ansprüche 5 bis 11 zur intramuskulären oder subkutanen Verabreichung.

15. Die Verwendung eines Dibuprenorphin-Dicarbonsäureester-Derivats nach einem der Ansprüche 1 bis 4 zur Herstellung eines injizierbaren Medikaments zum Bereitstellen einer verlängerten Analgesie bei einem Tier oder Mensch.

16. Die Verwendung einer Kombination aus (i) einem Buprenorphin-Monocarbonsäureester-Derivat, wie es in Teil (i) von Anspruch 5 definiert ist, oder einem Dibuprenorphin-Dicarbonsäureester-Derivat nach einem der Ansprüche 1 bis 4; und (ii) einem pharmazeutisch verträglichen Ölvehikel, zur Herstellung eines Medikaments zum Bereitstellen einer verlängerten Analgesie bei einem Tier oder Mensch.

17. Ein Verfahren zum Herstellen eines Dibuprenorphin-Dicarbonsäureester-Derivats mit Formel (II) nach Anspruch 1, das die Schritte umfasst:
(i') Behandeln von Buprenorphin-HCI oder -Base mit Trimethylamin in der Gegenwart von Methylenchlorid; und
(ii') Zugeben einer Verbindung mit Formel R₁(COOH)₂, oder einem Säureanhydrid oder einem Säurechlorid davon zu der Mischung von Schritt (i') in der Gegenwart von Methylenchlorid, wobei R₁ in der Formel R₁(COOH)₂ eine bivalente Komponente einer gesättigten oder ungesättigten aliphatischen Gruppe mit 1 bis 40 Kohlenstoffatomen ist, die optional mit einer Phenylgruppe substituiert ist.

18. Ein Verfahren nach Anspruch 17, wobei die in Schritt (ii') verwendete Verbindung R₁(COOH)₂ eine aliphatische Dicarbonsäure mit 30 bis 40 Kohlenstoffatomen oder ein Säureanhydrid oder ein Säurechlorid davon ist.

19. Ein Verfahren nach Anspruch 17, wobei Heptandioylchlorid oder Sebacoylsäure in Schritt (ii') verwendet wird.

## Revendications

1. Dérivé ester dicarboxylique de dibuprénorphine de formule (II): 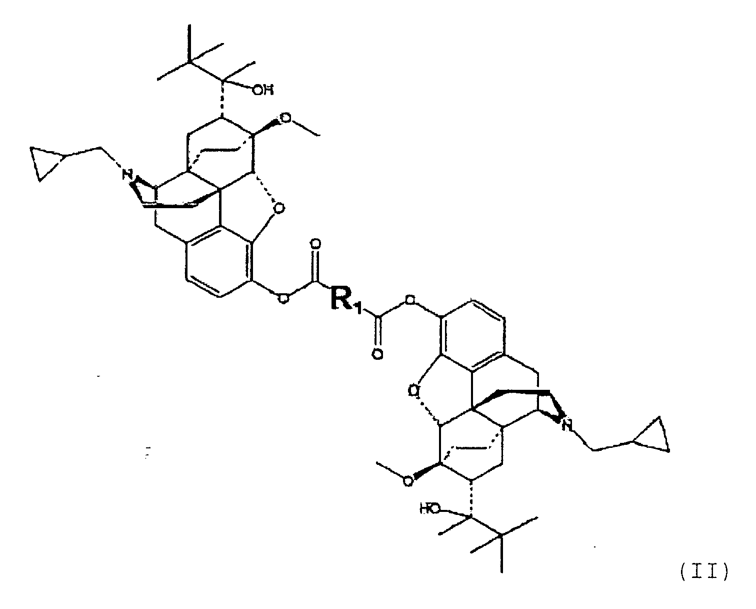 dans laquelle R₁ est un fragment divalent d'un groupe aliphatique saturé ou insaturé ayant de 1 à 40 atomes de carbone et éventuellement substitué par un groupe phényle.

2. Dérivé ester dicarboxylique de dibuprénorphine selon la revendication 1, dans lequel R₁ est un groupe alkylène ayant de 1 à 40 atomes de carbone.

3. Dérivé ester dicarboxylique de dibuprénorphine selon la revendication 2, dans lequel R₁ est un groupe alkylène ayant de 1 à 20 atomes de carbone.

4. Dérivé ester dicarboxylique de dibuprénorphine selon la revendication 1, qui est du pimélate de dibuprénorphine ou un ester sébacoylique de dibuprénorphine.

5. Composition pharmaceutique analgésique comprenant :
(a) une quantité thérapeutiquement efficace de :
(i) un dérivé ester monocarboxylique de buprénorphine de formule (I) : 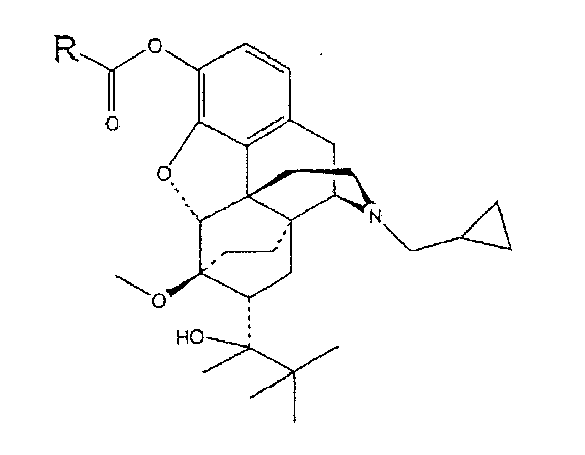 dans laquelle R est choisi parmi un groupe aliphatique saturé ou insaturé, à chaîne linéaire ou ramifiée, éventuellement substitué par un groupe aryle, ou un groupe aryle éventuellement substitué par un groupe aliphatique saturé ou insaturé à chaîne linéaire ou ramifiée ;
à condition que R ne soit pas choisi parmi un groupe méthyle, éthyle, propyle, n-butyle, n-pentyle, n-hexyle ou isopropyle ; ou
(ii) un dérivé ester dicarboxylique de dibuprénorphine selon l'une quelconque des revendications 1 à 4 ; et
(b) un véhicule huileux pharmaceutiquement acceptable.

6. Composition pharmaceutique analgésique selon la revendication 5, dans laquelle ledit véhicule est de l'huile de sésame, de l'huile de ricin, de l'huile de graines de coton, de l'huile de soja, de l'huile d'arachide ou l'ester éthylique d'huile d'arachide, ou une combinaison de ceux-ci.

7. Composition pharmaceutique analgésique selon la revendication 5 ou la revendication 6, comprenant un dérivé monoester de buprénorphine de formule (I) dans laquelle R est un groupe alkyle éventuellement substitué par un groupe phényle.

8. Composition pharmaceutique analgésique selon la revendication 5 ou la revendication 6, comprenant un dérivé ester monocarboxylique de buprénorphine de formule (I) dans laquelle R est un groupe alkyle ayant de 2 à 40 atomes de carbone.

9. Composition pharmaceutique analgésique selon la revendication 5 ou la revendication 6, comprenant un dérivé ester monocarboxylique de buprénorphine de formule (I) dans laquelle R est un groupe alkyle ayant de 5 à 20 atomes de carbone.

10. Composition pharmaceutique analgésique selon la revendication 5 ou la revendication 6, comprenant un dérivé ester monocarboxylique de buprénorphine de formule (I) dans laquelle R est choisi parmi un groupe alkyle à chaîne linéaire éventuellement substitué par un groupe phényle, un groupe alkyle ramifié éventuellement substitué par un groupe phényle, un groupe phényle éventuellement substitué par un groupe aliphatique à chaîne linéaire ou un groupe phényle éventuellement substitué par un groupe aliphatique ramifié.

11. Composition pharmaceutique analgésique selon la revendication 5 ou la revendication 6, comprenant un dérivé ester monocarboxylique de buprénorphine de formule (I), qui est du pivalate de buprénorphine, du benzoate de buprénorphine, du décanoate de buprénorphine ou du palmitate de buprénorphine.

12. Utilisation d'une composition comprenant une quantité thérapeutiquement efficace d'une base de buprénorphine et d'une huile injectable et sensiblement exempte d'eau pour la fabrication d'un médicament injectable pour fournir une analgésie prolongée à un humain ou à un animal.

13. Utilisation d'une composition selon la revendication 12, dans laquelle ledit véhicule huileux injectable est de l'huile de sésame, de l'huile de ricin, de l'huile de graines de coton, de l'huile de soja, de l'huile d'arachide ou l'ester éthylique d'huile d'arachide, ou une combinaison de ceux-ci.

14. Composition pharmaceutique analgésique selon l'une quelconque des revendications 5 à 11, qui est destinée à une administration intramusculaire ou sous-cutanée.

15. Utilisation d'un dérivé ester dicarboxylique de dibuprénorphine selon l'une quelconque des revendications 1 à 4 pour la préparation d'un médicament injectable pour fournir une analgésie prolongée à un animal ou à un humain.

16. Utilisation d'une combinaison de (i) un dérivé ester monocarboxylique de buprénorphine tel que défini dans la partie (i) de la revendication 5 ou d'un dérivé ester dicarboxylique de dibuprénorphine selon l'une quelconque des revendications 1 à 4 ; et (ii) un véhicule huileux pharmaceutiquement acceptable, pour la préparation d'un médicament pour fournir une analgésie prolongée à un animal ou à un humain.

17. Procédé de préparation d'un dérivé ester dicarboxylique de dibuprénorphine de formule (II) selon la revendication 1, comprenant les étapes consistant à :
(i') traiter du chlorhydrate ou une base de buprénorphine avec de la triméthylamine en présence de chlorure de méthylène ; et
(ii') ajouter au mélange de l'étape (i') un composé de formule R₁ (COOH)₂ ou un anhydride d'acide ou un chlorure d'acide de celui-ci, en présence de chlorure de méthylène, où R₁ dans la formule R₁ (COOH)₂ est un fragment divalent d'un groupe aliphatique saturé ou insaturé ayant de 1 à 40 atomes de carbone et éventuellement substitué par un groupe phényle.

18. Procédé selon la revendication 17, dans lequel le composé de R₁ (COOH)₂ utilisé dans l'étape (ii') est un acide dicarboxylique aliphatique ayant de 30 à 40 atomes de carbone ou un anhydride d'acide ou un chlorure d'acide de celui-ci.

19. Procédé selon la revendication 17, dans lequel on utilise du chlorure d'heptanedioyle ou de l'acide sébacoylique dans l'étape (ii').
